# EUROPÄISCHE PATENTANMELDUNG

(11) **EP 2 471 776 A1**
(43) Veröffentlichungstag der Anmeldung: **04.07.2012**
(21) Anmeldenummer: 10197155.4
(22) Anmeldetag: 28.12.2010
(51) Int. Cl.: C07D 213/73, C07D 213/74, C07D 213/75, A01N 43/40

(54) **Pyridin-2-ylpropandinitrile und deren Verwendung als Herbizide**

(71) Anmelder: Bayer CropScience AG, 40789 Monheim (DE)
(72) Erfinder: Brünjes, Marco, 65719 Hofheim (DE); Dietrich, Hansjörg, 65835 Liederbach am Taunus (DE); Döller, Uwe, 63110 Rodgau (DE); Hoffmann, Michael Gerhard, 65439 Flörsheim (DE); Häuser-Hahn, Isolde, 51375 Leverkusen (DE); Gatzweiler, Elmar, 61231 Bad Nauheim (DE); Rosinger, Christopher Hugh, 65719 Hofheim (DE); Heinemann, Ines, 65719 Hofheim (DE)

(57) **Zusammenfassung**

Pyridin-2-ylpropandinitrile, Verfahren zu deren Herstellung sowie deren Verwendung als Herbizide und Pflanzenwachstumsregulatoren

Die Erfindung betrifft Verbindungen der Formel (I) und deren N-Oxide, Salze und agrochemisch geeigneten Derivate

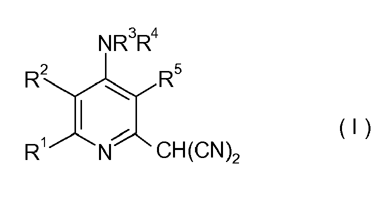

Verfahren zu deren Herstellung, sowie deren Verwendung als Herbizide und Pflanzenwachstumsregulatoren, insbesondere als Herbizide zur selektiven Bekämpfung von Schadpflanzen in Nutzpflanzenkulturen.

## Beschreibung

Die vorliegende Erfindung betrifft neue, herbizid wirksame Pyridin-2-ylpropandinitril-Derivate sowie Verfahren zu deren Herstellung. Weiterer Gegenstand der vorliegenden Erfindung ist deren Verwendung als Herbizid, insbesondere als Herbizid zur selektiven Bekämpfung von Schadpflanzen in Nutzpflanzenkulturen, und als Pflanzenwachstumsregulator allein oder in Kombination mit Safenern und/oder in Mischung mit anderen Herbiziden.

Aus dem Stand der Technik ist bekannt, dass substituierte Pyridin-Derivate herbizide bzw. schädlingsbekämpfende Eigenschaften besitzen (siehe beispielsweise WO 2001/51468, WO 2003/011853, WO 2006/062979, WO 2007/082098, WO 2007/092184, WO 2009/029518 oder WO 2009/046090). Die aus den oben genannten Schriften bekannten Wirkstoffe weisen bei ihrer Anwendung jedoch Nachteile auf, z. B. dass sie (a) keine oder aber eine nur unzureichende herbizide Wirkung gegen Schadpflanzen, (b) ein zu geringes Spektrum der bekämpften Schadpflanzen, oder (c) eine zu geringe Selektivität in Nutzpflanzenkulturen besitzen.

Es ist deshalb wünschenswert, chemische Wirkstoffe bereitzustellen, die mit Vorteilen als Herbizide oder Pflanzenwachstumsregulatoren eingesetzt werden können.

Es wurde nun überraschend gefunden, dass bestimmte substituierte Pyridin-2-ylpropandinitril-Derivate gute herbizide Wirkung und gleichzeitig hohe Verträglichkeit gegenüber Nutzpflanzen aufweisen. Ein Gegenstand der vorliegenden Erfindung sind daher Verbindungen der Formel (I), deren N-Oxide, Salze und agrochemisch geeigneten Derivate, 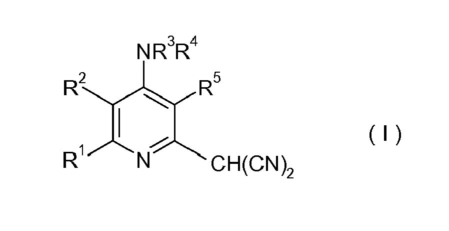 worin die Reste die folgende Bedeutung aufweisen:
- R¹: ist Phenyl, optional substituiert mit einem, zwei oder drei Resten R^{x}, welche unabhängig voneinander ausgewält sind aus Halogen, Cyano, Nitro, Amino, (C₁-C₆)Alkyl, (C₁-C₆)Haloalkyl, (C₁-C₄)Hydroxyalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₂-C₄)Alkenyloxy, (C₂-C₄)Haloalkenyloxy, (C₂-C₄)Alkinyloxy, (C₃-C₄)Haloalkinyloxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, (C₂-C₄)Alkenylthio, (C₂-C₄)Halo-alkenylthio, (C₂-C₄)Alkenylsulfinyl, (C₂-C₄)Haloalkenylsulfinyl, (C₂-C₄)Alkenyl-sulfonyl, (C₂-C₄)Haloalkenylsulfonyl, (C₂-C₄)Alkinylthio, (C₃-C₄)Haloalkinylthio, (C₃-C₄)Alkinylsulfinyl, (C₃-C₄)Haloalkinylsulfinyl, (C₃-C₄)Alkinylsulfonyl, (C₃-C₄)Haloalkinylsulfonyl, (C₁-C₆)Alkylamino, (C₂-C₈)Dialkylamino, (C₂-C₆)Alkylcarbonyl, (C₂-C₆)Alkoxycarbonyl, (C₂-C₆)Alkylaminocarbonyl, (C₃-C₈)Dialkylaminocarbonyl, (C₃-C₆)Trialkylsilyl, Phenyl, Phenoxy oder einen 5-oder 6-gliedrigen heteroaromatischen Ring, wobei jeder Phenyl-Ring, Phenoxy-Ring oder 5-oder 6-gliedrige heteroaromatische Ring optional substituiert sein kann mit einem, zwei oder drei Substituenten, die unabhängig voneinander ausgewählt sind aus R²⁸; oder wobei zwei benachbarte Reste R^{x} bilden gemeinsam eine -OCH₂O-, -CH₂CH₂O-, -OCH₂CH₂O-, -OCH(CH₃)O-, -OC(CH₃)₂O-, -OCF₂O-, -CF₂CF₂O-, -OCF₂CF₂O- oder -CH=CH-CH=CH- Gruppe bilden können; oder
(C₁-C₆)-Alkyl, optional substituiert mit einem, zwei oder drei Resten R^{y}, welche unabhängig voneinander ausgewählt sind aus Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio; oder
(C₂-C₆)-Alkenyl, optional substituiert mit einem, zwei oder drei Resten R^{z}, welche unabhängig voneinander ausgewählt sind aus Halogen, (C₁-C₆)Alkyl, (C₁-C₆)Haloalkyl, (C₁-C₃)Alkoxy, (C₁-C₂)Haloalkoxy, (C₁-C₃)Alkylthio oder (C₁-C₂)Haloalkylthio;
- R²: ist ausgewählt aus Wasserstoff, Fluor, Chlor, Brom, lod, Cyano, Nitro, Hydroxy (C₁-C₄)Alkyl, (C₂-C₄)Alkenyl, (C₂-C₄)Alkinyl, (C₁-C₄)Alkoxy, (C₁-C₄)Haloalkyl, (C₁-C₄)Haloalkoxy, SR⁶ oder N(R⁷)R⁸; worin R⁶ ausgewählt ist aus Wasserstoff, (C₁-C₄)Alkyl oder (C₁-C₃)Haloalkyl und R⁷ und R⁸ unabhängig voneinander ausgewählt sind aus Wasserstoff oder (C₁-C₄)Alkyl;
- R³: ist ausgewählt aus Wasserstoff, (C₁-C₄)Alkyl optional substituiert mit einem oder zwei Resten R⁹, (C₂-C₄)Alkenyl optional substituiert mit einem oder zwei Resten R¹⁰, oder (C₂-C₄)Alkinyl optional substituiert mit einem oder zwei Resten R¹¹; oder R³ ist C(=O)R¹², NO₂, OR¹³, S(O)₂R¹⁴, N(R¹⁵)R¹⁶ oder N=C(R¹⁷)R¹⁸;
- R⁴: ist ausgewählt aus Wasserstoff, (C₁-C₄)Alkyl, optional substituiert mit einem oder zwei Resten R⁹, oder C(=O)R¹²;
oder
- R³ und R⁴: bilden gemeinsam eine -(CH₂)₄-, -(CH₂)₅-, -CH₂CH=CHCH₂- oder -(CH₂)₂O(CH₂)₂- Gruppe, wobei jede der Gruppen optional substituiert sein kann mit einem oder zwei Resten R¹⁹; oder R³ und R⁴ gemeinsam eine =C(R²⁰)N(R²¹)R²² oder =C(R²³)OR²⁴ Gruppe bilden;
dabei ist jeder Rest R⁹, R¹⁰ und R¹¹ unabhängig voneinander Halogen, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
- R⁵: ist ausgewählt aus Wasserstoff, Fluor, Chlor, Brom, lod, Cyano, Nitro, Hydroxy, (C₁-C₄)Alkyl, (C₂-C₄)Alkenyl, (C₂-C₄)Alkinyl, (C₁-C₄)Alkoxy, (C₁-C₄)Haloalkyl, (C₁-C₄)Haloalkoxy, (C₁-C₄)Alkylthio, (C₁-C₄)Haloalkylthio, (C₁-C₄)Alkylamino, (C₁-C₄)Dialkylamino,
- R¹²: ist ggf. unabhängig von weiteren Resten R¹² ausgewählt aus Wasserstoff, (C₁-C₄)Alkyl, (C₁-C₃)Haloalkyl, (C₁-C₄)Alkoxy, Phenyl, Phenoxy oder Benzyloxy;
- R¹³: ist Wasserstoff, (C₁-C₄)Alkyl, (C₁-C₃)Haloalkyl oder CHR²⁵C(O)OR²⁶;
- R¹⁴: ist (C₁-C₄)Alkyl oder (C₁-C₃)Haloalkyl;
- R¹⁵: ist Wasserstoff, (C₁-C₄)Alkyl oder C(=O)R²⁷;
- R¹⁶: ist Wasserstoff oder (C₁-C₄)Alkyl;
- R¹⁷: ist Wasserstoff, (C₁-C₄)Alkyl oder Phenyl optional substituiert mit einem, zwei oder drei Resten, welche voneinander unabhängig ausgewählt sind aus CH₃, Cl oder OCH₃,
- R¹⁸: ist Wasserstoff oder (C₁-C₄)Alkyl; oder R¹⁷ und R¹⁸ bilden gemeinsam eine -(CH₂)₄- oder -(CH₂)₅- Gruppe;
- R¹⁹: ggf. unabhängig von weiteren Resten R¹⁹ ausgewählt aus Halogen, (C₁-C₃)Alkyl, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
- R²⁰: ist Wasserstoff oder (C₁-C₄)Alkyl;
- R²¹ und R²²: sind unabhängig voneinander ausgewählt aus Wasserstoff und (C₁-C₄)Alkyl; oder R²¹ und R²² bilden gemeinsam eine -(CH₂)₄-, -(CH₂)₅-, -CH₂CH=CHCH₂- oder -(CH₂)₂O(CH₂)₂- Gruppe;
- R²³: ist Wasserstoff oder (C₁-C₄)Alkyl;
- R²⁴: ist (C₁-C₄)Alkyl;
- R²⁵: ist Wasserstoff, (C₁-C₄)Alkyl, (C₁-C₃)Haloalkyl, (C₁-C₄)Alkoxy, Phenyl, Phenoxy oder Benzyloxy;
- R²⁶: ist Wasserstoff, (C₁-C₄)Alkyl oder (C₁-C₄)Alkoxy;
- R²⁷: ist Wasserstoff, C₁-C₄ Alkyl oder Benzyl; und
- R²⁸: ist ggf. unabhängig von weiteren Resten R²⁸ ausgewählt aus Halogen, Cyano, Nitro, (C₁-C₆)Alkyl, (C₁-C₆)Haloalkyl, (C₂-C₄)Alkenyl, (C₂-C₄)Haloalkenyl, (C₃-C₄)Alkinyl, (C₃-C₄)Haloalkinyl, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₄)Alkylamino, (C₂-C₈)Dialkylamino, (C₂-C₄)Alkylcarbonyl, (C₂-C₆)Alkoxycarbonyl, (C₂-C₆)Alkylaminocarbonyl, (C₃-C₈)Dialkylaminocarbonyl oder (C₃-C₆)Trialkylsilyl.

Dabei ist bei den genannten Resten stets die Gesamtzahl an C-Atomen angegeben, z.B. ist ein Dimethylaminocarbonyl-Rest ein C₃-Rest.

Bevorzugt sind Verbindungen der Formel (I), worin die Reste folgende Bedeutung aufweisen:
- R¹: ist Phenyl, optional substituiert mit einem, zwei oder drei Resten R^{x}, welche unabhängig voneinander ausgewählt sind aus Halogen, Cyano, Nitro, Methyl, Ethyl, n-Propyl, i-Propyl, n-Butyl, t-Butyl, Cyclopropyl, Cyclobutyl, Cyclohexyl, , Difluormethyl, Trifluormethyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₆)Alkylamino, (C₂-C₈)Dialkylamino, oder wobei zwei benachbarte Reste R^{x} bilden gemeinsam eine -OCH₂O-, - CH₂CH₂O-, -OCH₂CH₂O-, -OCH(CH₃)O-, -OC(CH₃)₂O-, -OCF₂O-, -CF₂CF₂O- oder -OCF₂CF₂O- Gruppe bilden können; oder
(C₁-C₆)Alkyl, optional substituiert mit einem, zwei oder drei Resten R^{y}, welche unabhängig voneinander ausgewählt sind aus Fluor, Chlor, Brom, Iod, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio; oder
(C₂-C₆)Alkenyl, optional substituiert mit einem, zwei oder drei Resten R^{z}, welche unabhängig voneinander ausgewählt sind aus Fluor, Chlor, Brom, Iod,, Methoxy, Ethoxy, n-Propoxy, i-Propoxy, n-Butoxy, t-Butoxy, (C₁-C₂)Haloalkoxy, (C₁-C₃)Alkylthio oder (C₁-C₂)Haloalkylthio;
- R²: ist Wasserstoff, Fluor, Chlor, Brom, Iod, Cyano, Methyl, Ethyl, Vinyl, Methoxy, Ethoxy;
- R³: ist Wasserstoff, Methyl, Ethyl, n-Propyl, i-Propyl, n-Butyl, t-Butyl, (C₂-C₄)Alkenyl, (C₂-C₄)Alkinyl, C(=O)R¹², OR¹³, N(R¹⁵)R¹⁶ oder N=C(R¹⁷)R¹⁸;
- R⁴: ist Wasserstoff oder Methyl, Ethyl, n-Propyl, i-Propyl, n-Butyl, t-Butyl, optional substituiert mit einem oder zwei Resten R⁹, oder C(=O)R¹²; oder
- R³ und R⁴: bilden gemeinsam eine Gruppe -(CH₂)₄-, -(CH₂)₅-, -(CH₂)₂O(CH₂)₂- oder =C(R²⁰)N(R²¹)R²²;
dabei ist jeder Rest R⁹ unabhängig voneinander ausgewählt aus Fluor, Chlor, Brom, Iod,, Methoxy, Ethoxy, n-Propoxy, i-Propoxy, Difluormethyl, Trifluormethyl" (C₁-C₃)Alkylthio, (C₁-C₃)Haloalkylthio, Amino, Methylamino, Ethylamino, n-Propylamino, i-Propylamino, Dimethylamino, Diethylamino, Methyl-Ethylamino oder (C₂-C₄)Alkoxycarbonyl;
- R⁵: ist Wasserstoff, Fluor, Chlor, Brom, Iod, Cyano, Methyl, Ethyl, n-Propyl, i-Propyl, n-Butyl, t-Butyl Methoxy, Ethoxy, n-Propoxy, i-Propoxy, n-Butoxy, t-Butoxy, (C₁-C₄)Alkylthio,
- R¹²: ist Wasserstoff, Methyl, Ethyl, n-Propyl, i-Propyl, n-Butyl, t-Butyl, Difluormethyl, Trifluormethyl, Methoxy, Ethoxy, n-Propoxy, i-Propoxy, n-Butoxy, t-Butoxy, Phenyl, Phenoxy oder Benzyloxy;
- R¹³: ist Wasserstoff, Methyl, Ethyl, n-Propyl, i-Propyl, n-Butyl, t-Butyl, Difluormethyl, Trifluormethyl, oder CHR²⁵C(O)OR²⁶;
- R¹⁵: ist Wasserstoff, (C₁-C₄)Alkyl oder C(=O)R²⁷;
- R¹⁶: ist Wasserstoff oder Methyl, Ethyl, n-Propyl, i-Propyl, n-Butyl, t-Butyl;
- R¹⁷: ist Wasserstoff, Methyl, Ethyl, n-Propyl, i-Propyl, n-Butyl, t-Butyl oder Phenyl optional substituiert mit einem, zwei oder drei Resten, welche voneinander unabhängig ausgewählt sind aus CH₃, Cl oder OCH_{3;}
- R¹⁸: ist Wasserstoff oder Methyl, Ethyl, n-Propyl, i-Propyl, n-Butyl, t-Butyl; oder R¹⁷ und R¹⁸ bilden gemeinsam eine -(CH₂)₄- oder -(CH₂)₅- Gruppe;
- R²⁰: ist Wasserstoff oder Methyl, Ethyl, n-Propyl, i-Propyl, n-Butyl, t-Butyl;
- R²¹ und R²²: sind unabhängig voneinander Wasserstoff oder Methyl, Ethyl, n-Propyl, i-Propyl, n-Butyl, t-Butyl; oder R²¹ and R²² bilden gemeinsam eine -(CH₂)₄-, - (CH₂)₅-, -CH₂CH=CHCH₂- oder -(CH₂)₂O(CH₂)₂- Gruppe;
- R²⁵: ist Wasserstoff, Methyl, Ethyl, n-Propyl, i-Propyl, n-Butyl, t-Butyl, Difluormethyl, Trifluormethyl, (C₁-C₄)Alkoxy, Phenyl, Phenoxy oder Benzyloxy; und
- R²⁶: ist Wasserstoff, Methyl, Ethyl, n-Propyl, i-Propyl, n-Butyl, t-Butyloder Methoxy, Ethoxy, n-Propoxy, i-Propoxy, n-Butoxy, t-Butoxy.

Besonders bevorzugt sind Verbindungen der Formel (I), worin die Reste folgende Bedeutung aufweisen:
- R¹: ist Phenyl, optional substituiert mit einem, zwei oder drei Resten R^{x}, welche unabhängig voneinander ausgewählt sind aus Fluor, Chlor, Brom, Iod, Cyano, Nitro, Methylthio, Methyl, Ethyl, oder wobei zwei benachbarte Reste R^{x} eine gemeinsame OCH₂O-, -OCH₂CH₂O- oder -OCH(CH₃)O- Gruppe bilden können; oder Hexyl, Isopropyl, tert-Butyl, Cyclopropyl, Cyclobutyl, Cyclohexyl, Trifluormethyl, Trifluormethoxy, Methoxy, Ethoxy, (C₁-C₄)Haloalkoxy, Dimethylamino, Diethylamino, Dimethylamino, Trifluormethylsulfinyl, Methylsulfonyl, 4-Chlorbenzyl, Vinyl, Vinylphenyl, Cyclopropylvinyl, Methylvinyl;
- R²: ist Wasserstoff, Fluor, Chlor, Brom, Iod, Methyl, Ethyl, Vinyl, Methoxy oder Cyano;
- R³: ist Wasserstoff, Methyl, Ethyl;
- R⁴: ist Wasserstoff, Methyl, Ethyl oder Acetyl ; oder
R³ und R⁴ bilden gemeinsam eine =CHN(Me)₂ Gruppe
- R⁵: ist Wasserstoff, F, Cl, Br, Methyl, Ethyl, Cyano, (C₁-C₄)Alkoxy, (C₁-C₄)Alkylthio.

Die Verbindungen der Formel (I) können durch Wasserstoffverschiebung Tautomere bilden, welche ebenfalls Gegenstand der vorliegenden Erfindung sind. So können beispielsweise die Pyridin-2-ylpropandinitril-Derivate (I) auch
als Pyridin-2(1H)-ylidenpropandinitrile (la) vorliegen, wobei erfindungsgemäß sämtliche Tautomere umfasst sind, insbesondere aber die Formen (I) und (Ia).

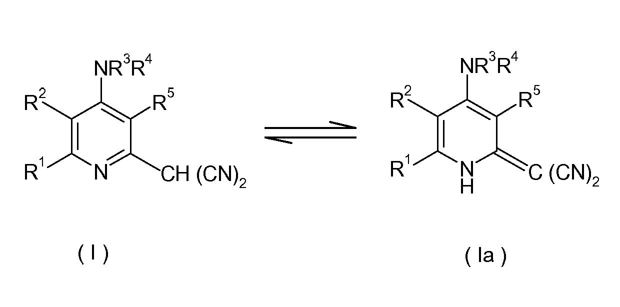

Die Verbindungen der Formel (I) können je nach Art und Verknüpfung der Substituenten als Stereoisomere vorliegen. Die durch ihre spezifische Raumform definierten möglichen Stereoisomeren, wie Enantiomere, Diastereomere, Z- und E-Isomere, geometrische Isomere und Atropisomere und deren Gemische sind alle von Formel (I) umfasst.

Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome ( = asymmetrisch substituierte Kohlenstoffatome) vorhanden und/oder asymmetrische Schwefelatome in Form von Sulfoxiden, die in zwei enantiomeren Formen existieren können, so können Enantiomere und Diastereomere auftreten. Stereoisomere lassen sich aus den bei der Herstellung anfallenden Gemischen nach üblichen Trennmethoden, beispielsweise durch chromatographische Trennverfahren, isolieren. Ebenso können Stereoisomere durch Einsatz stereoselektiver Reaktionen unter Verwendung optisch aktiver Ausgangsund/oder Hilfsstoffe selektiv hergestellt werden. Die Erfindung betrifft somit auch alle Stereoisomeren, die von der allgemeinen Formel (I) umfasst werden, auch wenn sie nicht mit ihrer spezifischen Stereoform angegeben sind, und deren Gemische.

Die Verbindungen der Formel (I) können agrochemisch geeignete Salze bilden. Salzbildung kann in bekannter Weise, z. B. durch Einwirkung einer Base auf solche Verbindungen der Formel (I) erfolgen, die ein acides Wasserstoffatom tragen, z.B. im Falle dass R¹ eine OH-Gruppe oder eine Sulfonamid-Gruppe -NHSO₂- enthält. Eine Salzbildung kann ebenfalls durch das Einwirken einer Base auf CH-acide Verbindungen, wie (substituierte) Propandinitrile, erfolgen. Geeignete Basen sind beispielsweise organische Amine, wie Trialkylamine, Morpholin, Piperidin oder Pyridin sowie Ammoniak, Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natriumund Kaliumsalze, oder auch Salze mit organischen Aminen oder Ammoniumsalze, zum Beispiel mit Ammoniumionen der Formel [NRR'R"R"']⁺, worin R, R', R" und R"' jeweils unabhängig voneinander H oder einen organischen Rest, insbesondere (C₁-C₆)Alkyl, (C₆-C₁₀)Aryl, (C₇-C₂₀)Aralkyl oder (C₇-C₂₀)Alkylaryl darstellen. Beispiele sind [NH₄]⁺, [NH₃CH₃]⁺, [NH₂(CH₃)₂]⁺ [NH(CH₃)₃]⁺, [N(CH₃)₄]⁺, [NH₂CH₃C₂H₅]⁺ oder [NH₂CH₃C₆H₅]⁺. In Frage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure, oder Sulfonsäuren, wie zum Beispiel p-Toluolsulfonsäure, an eine basische Gruppe, wie Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, Carbonsäuren oder CH-acide Gruppen, wie Propandinitrile, vorliegen, können auch innere Salze mit ihrerseits protonierbaren Gruppen, wie Aminogruppen, bilden.

Die Verbindungen der Formel (I) können N-Oxide bilden. Die N-Oxide können in bekannter Weise z. B. durch Oxidation der jeweiligen Pyridine mit Peroxycarbonsäuren oder Wasserstoffperoxid in Lösungsmitteln wie Acetonitril, Dichlormethan, Chloroform, Aceton, DMF, Essigsäure, z. B. bei Temperaturen zwischen 0° und 100°C gebildet werden (siehe: D. Spitzner, Science of Synthesis 2004, 15, 218 und die jeweils darin zitierte Literatur).

Die Verbindungen der Formel (I) und deren N-Oxide, Salze und agrochemisch geeigneten Salze werden auch kurz als erfindungsgemäß verwendete oder erfindungsgemäße Verbindungen bezeichnet. Die vorstehend und weiter unten verwendeten Bezeichnungen sind dem Fachmann geläufig und haben insbesondere die im Folgenden erläuterten Bedeutungen:
Der Begriff *"agrochemisch geeignete Derivate",* der zur Beschreibung der erfindungsgemäßen Variationen der Aminogruppen in 3-Position verwendet wird, bezeichnet auch jegliche Silylamine, Phosphorylamine, Phosphinimine, Phosphoramidate, Sulfonamide, Sulfilimide, Sulfoximine, Aminale, Hemiaminale, Amide, Thioamide, Carbamate, Thiocarbamate, Amidine, Harnstoffe, Imine, Nitro, Nitroso, Azido oder sonstige stickstoffhaltige Derivate, die im Stand der Technik beschrieben sind und die
   (a) die herbizide Aktivität der Wirkstoffe nicht wesentlich beeinträchtigen und
   (b) in der Pflanze oder im Boden zu den freien Aminen der Formel (I) hydrolysiert, oxidiert oder metabolisiert werden oder werden können, die in Abhängigkeit vom pH-Wert in dissoziierter oder nicht-dissoziierter Form vorliegen.

Bevorzugte *agrochemisch geeignete Derivate* im Hinblick auf die Aminogruppen sind daher Salze, Amide, Sulfonamide und Carbamate.

Ein anorganischer Rest ist ein Rest ohne Kohlenstoffatome, vorzugsweise Halogen, OH und dessen anorganische Salze, bei denen das H durch ein Kation, beispielsweise Alkalimetall- und Erdalkalimetallsalze ersetzt wird, -NH₂ und dessen Ammoniumsalze mit (anorganischen) Säuren, beispielsweise Mineralsäuren, -N₃ (Azid), -N₂⁺A⁻ (Diazonium-Gruppe, wobei A- ein Anion darstellt), -NO, -NHOH, -NHNH₂, -NO₂, -ONO, -ONO₂, -SH, -SOH (Sulfensäure-Gruppe), -S(O)OH (Sulfinsäure-Gruppe), -S(O)₂OH (oder auch kurz SO₃H, Sulfonsäure-Gruppe), -O-SO₂H (Sulfit-Gruppe), -O-SO₃H (Sulfat-Gruppe), -SO₂NH₂ (Sulfamoyl-Gruppe), -SO₂NHOH (Hydroxysulfamoyl-Gruppe), -NHS(O)OH (Sulfinoamino-Gruppe), -NHS(O)₂OH (Sulfoamino-Gruppe), -P(O)(OH)₂ (Phosphonsäure-Gruppe), -O-P(OH)₃ (Phosphat-Gruppe), -P(O)(NH₂)₂, -PO(OH)(NH₂), -PS(OH)₂, -PS(NH₂)₂ oder -PS(OH)(NH₂), -B(OH)₂ (Boronsäure-Gruppe) und die hydratisierten oder dehydratisierten Formen der Säuregruppen sowie deren (anorganischen) Salze;
der Begriff "anorganischer Rest" umfasst auch den Wasserstoffrest (das Wasserstoffatom), wobei dieser in den Definitionen oft bereits Bestandteil des unsubstituierten Grundkörpers eines organischen Restes ist (Beispiel "unsubstituiertes Phenyl");
der Begriff "anorganischer Rest" umfasst hier vorzugsweise nicht Pseudohalogen-Gruppen wie CN, SCN, organische Metallkomplexe, Carbonat oder COOH, die wegen des Gehalts an C-Atomen den organischen Resten zugeordnet werden.

Die Bezeichnung "Halogen" oder "Halogenatom" bedeutet beispielsweise Fluor, Chlor, Brom oder Iod.

Wird die Bezeichnung für einen Rest verwendet, dann bedeutet "Halogen" oder "Halogenatom" beispielsweise ein Fluor-, Chlor-, Brom- oder lodatom.

Alkyl bedeutet einen geradkettigen, verzweigten oder cyclischen Kohlenwasserstoffrest. Der Ausdruck "(C₁-C₄)Alkyl" bedeutet beispielsweise eine Kurzschreibweise für Alkyl mit einem bis 4 Kohlenstoffatomen entsprechend der Bereichsangabe für C-Atome und umfasst z.B. die Reste Methyl, Ethyl, 1-Propyl, 2-Propyl, 1-Butyl, 2-Butyl, 2-Methylpropyl, tert-Butyl, Cyclopropyl und Cyclobutyl. Allgemeine Alkylreste mit einem größeren angegebenen Bereich von C-Atomen, z. B. "(C₁-C₆)Alkyl", umfassen entsprechend auch gradkettige, verzweigte oder cyclische Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, Cyclo-, n- oder i-Propyl, Cyclo-, n-, i-, t- oder 2-Butyl, Pentyle, Hexyle, wie Cyclohexyl, n-Hexyl, i-Hexyl und 1,3-Dimethylbutyl, Heptyle, wie Cycloheptyl, n-Heptyl, 1-Methylhexyl und 1,4-Dimethylpentyl.

Bevorzugte cyclische Alkylreste weisen vorzugsweise 3-8 Ring-C-Atome auf, z.B. Cyclopropyl, Cyclobutyl, Cyclopentyl oder Cyclohexyl. Im Falle von gegebenenfalls substituierten cyclischen Alkylresten werden cyclische Systeme mit Substituenten umfasst, wobei auch Substituenten mit einer Doppelbindung am cyclischen Alkylrest, z. B. eine Alkylidengruppe wie Methyliden, umfasst sind.

Im Falle von gegebenenfalls substituierten cyclischen Alkylresten werden auch mehrcyclische aliphatische Systeme umfaßt, wie Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von gegebenenfalls substituierten cyclischen Alkylresten werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Alkenyl- und Alkinylreste haben die Bedeutung der den Alkylresten entsprechenden möglichen ungesättigten geradkettigen, verzweigten oder cyclischen Reste, wobei mindestens eine Doppelbindung bzw. Dreifachbindung enthalten ist. Bevorzugt sind Reste mit einer Doppelbindung bzw. mit einer Dreifachbindung.

Alkenyl schließt auch geradkettige, verzweigte oder cyclische Kohlenwasserstoffreste mit mehr als einer Doppelbindung ein, wie 1,3-Butadienyl, 1,4-Pentadienyl oder Cyclohexadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl.

Alkinyl schließt auch geradkettige, verzweigte oder cyclische Kohlenwasserstoffreste mit mehr als einer Dreifachbindung oder auch mit einer oder mehreren Dreifachbindungen und einer oder mehreren Doppelbindungen ein, wie beispielsweise 1,3-Butatdienyl bzw. 3-Penten-1-in-1-yl.

Alkenyl bedeutet z.B. Vinyl, welches ggf. durch weitere Alkylreste substituiert sein kann, z . B . P r o p-1-en-1-yl, But-1-en-1-yl;Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, 2-Methylprop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl.

(C₂-C₆) -Alkinyl bedeutet beispielsweise Ethinyl, Propargyl, 1-Methyl-prop-2-in-1-yl, 2-Butinyl, 2-Pentinyl oder 2-Hexinyl, vorzugsweise Propargyl, But-2-in-1-yl, But-3-in-1-yl oder 1-Methyl-but-3-in-1-yl.

Cyclische Alkenylreste bedeuten ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituierte cyclische Alkylreste entsprechend.

Alkyliden, z. B. auch in der Form (C₁-C₁₀)Alkyliden, bedeutet den Rest eines geradkettigen, verzweigten oder cyclischen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Im Falle gegebenenfalls substituierten Aryls sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist Aryl in der Regel auch von dem Begriff "gegebenenfalls substituiertes Phenyl" umfasst.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes cyclisches Alkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkyl-aminoalkyl und Hydroxy-alkyl bedeuten;
im Begriff "substituierte Reste" wie substituiertes Alkyl (z.B. gradkettiges, verzweigtes oder cyclisches Alkyl) etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, SF₅, Aminosulfonyl, Alkyl, Alkenyl, Alkinyl, Monoalkyl-amino, Dialkyl-amino, N-Alkanoyl-amino, Alkoxy, Alkenyloxy, Alkinyloxy, Alkoxy-carbonyl, Alkenyloxy-carbonyl, Alkinyloxy-carbonyl, Aryloxycarbonyl, Alkanoyl, Alkenylcarbonyl, Alkinyl-carbonyl, Aryl-carbonyl, Alkylthio, Alkenylthio, Alkinylthio, Alkylsulfenyl, Alkylsulfinyl, wobei beide Enantiomere der Alkylsulfinylgruppe umfasst sind, Alkylsulfonyl, Monoalkyl-aminosulfonyl, Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-aminocarbonyl, N-Alkanoyl-aminocarbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl. Substituenten, die aus mehreren Substituentenebenen zusammengesetzt sind, sind bevorzugt beispielsweise Alkoxyalkyl, wie Monoalkoxyalkyl oder Dialkoxyalkyl, Alkylthioalkyl, Alkylthioalkoxy, Alkoxyalkoxy, wie Monoalkoxyalkoxy oder Dialkoxyalkoxy, Benzyl, Phenethyl, Benzyloxy, Haloalkyl, Haloalkoxy, Haloalkylthio, Haloalkanoyl, Haloalkylcarbonyl, Haloalkoxycarbonyl, Haloalkoxyalkoxy, Haloalkoxyalkylthio, Haloalkoxyalkanoyl, Haloalkoxyalkyl, Alkanoylalkyl, Haloalkanoylalkyl, Alkanoyloxyalkyl. Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl N-substituiert sind; vorzugsweise Mono- und Dialkyl-amino, Mono- und Diarylamino, wie gegebenenfalls substituierte Aniline, Acylamino, N,N-diacylamino, N-Alkyl-N-arylamino, N-Alkyl-N-acylamino sowie gesättigte N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Substituiertes Amino schließt auch quartäre Ammoniumverbindungen (Salze) mit vier organischen Substituenten am Stickstoffatom ein.

Eine der Carboxygruppe äquivalente Gruppe bedeutet beispielsweise ein Alkylester, Arylester, O-Alkylthioester, S-Alkyldithioester, S-Alkylthioester, Carboximidester, Carboximidthioester; 5,6-Dihydro-1,2,4-dioxazin-3-yl; 5,6-Dihydro-1,2,4-oxathiazin-3-yl, Trialkylorthoester, Dialkoxyalkylaminoester, Dialkylaminoalkoxyester, Trialkylaminoester, Amidine, Dialkoxyketenacetale oder Dialkyldithioketenacetale. Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Haogenalkyl, (C₁-C₄)Halogenalkoxy, (C₁-C₄)Alkylthio, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Ein organischer Säurerest bedeutet einen Rest einer Oxosäure oder Thiosäure der allgemeinen Formel

Rₖ E(=Q)p (OH) (QR')ₙ

wobei
- R: ein organischer Rest,
- E: ein Atom aus der Gruppe C, S, P,
- Q: unabhängig voneinander ein Atom oder ein Molekülfragment aus der Gruppe O, S, NR' und
- R': unabhängig voneinander ein Wasserstoffatom, Alkyl, Haloalkyl, Alkoxyalkyl oder gegebenenfalls Aryl bedeutet.
- *k, p*: sind natürliche Zahlen, *k* = 1, 2; *p* = 0 - 2;
- *n*: ist eine natürliche Zahl oder Null.

Der organische Säurerest entsteht formal durch Abtrennen einer Hydroxygruppe an der Säurefunktion, wobei der organische Rest R in der Säure auch über ein oder mehrere Heteroatome mit der Säurefunktion verbunden sein kann:

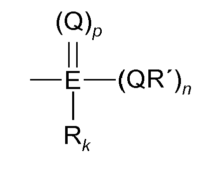

Für Oxosäuren des Kohlenstoffs ist dies im IUPAC Compendium of Chemical Terminology (1997) beschrieben.

Beispiele für organische Säurereste, die von den Oxosäuren bzw. Thiosäuren des Schwefels abgeleitet sind (E = S), sind S(O)OCH₃, SO₂OH, SO₂OCH₃ oder SO₂NHR (N-substituierte Sulfonamidsäuren).

Im Falle von *k* = 1 sind auch Alkylsulfonyl- und Alkylsulfinyl-Reste, wie z.B. (H₃C)S(O)₂, (F₃C)S(O)₂, p-TolylS(O)₂, (H₃C)S(O)(NH-n-C₄H₉), (C₆H₅)S(S)(O) oder (C₆H₅)S(O) mit umfasst.

Beispiele für organische Säurereste, die von den Oxosäuren bzw. Thiosäuren des Phosphors abgeleitet sind (E = P), sind von der Phosphinsäure und der Phosphonsäure abgeleitete Reste, wobei diese Reste weiter verestert sein können, z.B. -PO(OCH₃)₂, (C₂H₅O)P(O)OH, (C₂H₅O)P(O)(SC₆H₅), (H₃CO)P(O)NH(C₆H₅) oder -PO(NMe₂)₂.

Im Falle von *k* = 1 sind auch Alkylphosphinyl- und Alkylphosphonyl-Reste, wie z.B. (H₃C)₂ P(O), (C₆H₅)₂P(O), (H₃C)(C₆H₅)P(O); (H₃C)P(O)OCH₃, (H₅C₂)P(O)(OC₂H₅), (C₆H₅)P(O)(OC₂H₅), (C₂H₅)P(O)(SC₆H₅), (H₃C)P(O)NH(C₆H₅), (H₃C)P(S)(NH-i-C₃H₇), (C₆H₅)P(S)(OC₂H₅) oder (C₆H₅)P(S)(SC₂H₅) mit umfasst.

Organische Säurereste, die von den Oxosäuren des Kohlenstoffs abgeleitet sind (E = C, Q = O), werden im engeren Sinne auch mit dem Begriff "Acyl" bezeichnet.

Beispiele für Acyl sind der Rest -CO-R einer Carbonsäure HO-CO-R und Reste davon abgeleiteter Säuren oder der Rest von Kohlensäuremonoestern oder N-substituierter Carbaminsäuren sowie Carbonate und deren Ester.

Acyl bedeutet beispielsweise Formyl, Oxalyl(ester), Alkylcarbonyl wie [(C₁-C₄)Alkyl]-carbonyl, Haloalkylcarbonyl, Phenylcarbonyl, Alkyloxycarbonyl, speziell tert.-Butyloxycarbonyl, Phenyloxycarbonyl, Benzyloxycarbonyl, Fluorenyloxycarbonyl, N-Alkyl-1-iminoalkyl, N-Alkyl- und N,N-Dialkylcarbamoyl. Dabei können die Reste jeweils im Alkyl- oder Phenylteil noch weiter substituiert sein, beispielsweise im Alkylteil durch ein oder mehrere Reste aus der Gruppe Halogen, Cyano, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d.h. einen Rest einer organischen Säure, bei der die Säuregruppe direkt mit dem C-Atom eines organischen Restes verbunden ist, beispielsweise Alkanoyl, wie Formyl und Acetyl, Aroyl wie Phenylcarbonyl, und andere Reste von gesättigten oder ungesättigten organischen Säuren.

"Aroyl" bedeutet einen wie vorstehend definierter Arylrest, der über eine CarbonylGruppe gebunden ist, z.B. die Benzoyl-Gruppe.

Wenn ein allgemeiner Rest mit "Wasserstoff' definiert ist, bedeutet dies ein Wasserstoffatom.

Mit "yl-Position" eines Restes ist dessen Bindungstelle bezeichnet.

Gegenstand der vorliegenden Erfindung sind auch Methoden zur Herstellung der erfindungsgemäßen Verbindungen. Die erfindungsgemäßen Verbindungen können alternativ durch verschiedene Verfahren dargestellt werden.

In den nachfolgenden Verfahren werden partiell Lösemittel verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen.

Verbindungen der Formel (I) können z.B. durch Reaktion der entsprechenden Verbindungen der Formel (II) mit dem jeweiligen Alkalisalz des Malondinitrils (III) erhalten werden. In den Verbindungen (II) stellt X eine geeignete Abgangsgruppe wie z. B. Chlor, Brom, Iod oder Methansulfonyl dar. Die jeweiligen Salze des Malondinitrils können z. B. in situ durch Behandlung von Malondinitril (III) mit verschiedenen Basen wie z. B. n-Butyllithium, Lithiumdiisopropylamid, Natriumhydrid oder Kaliumcarbonat bei Temperaturen zwischen -80°C und 80°C generiert werden. Nach Zugabe der jeweiligen Verbindung (II) wird die Reaktion in einem Temperaturbereich zwischen 0°C und 130°C durchgeführt, bevorzugt in aprotischen Lösungsmitteln wie THF, DMSO, DMF oder Dioxan.

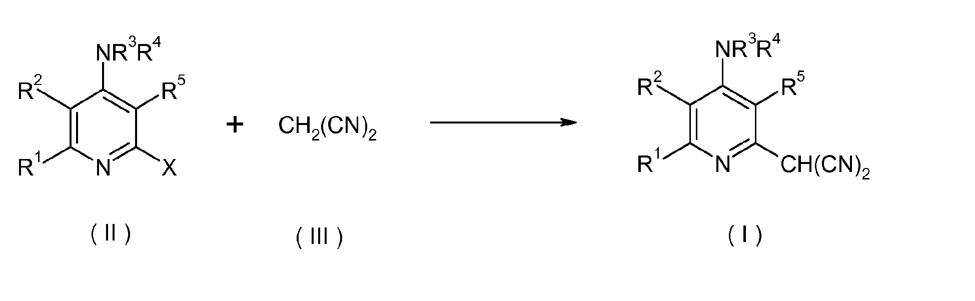

Verbindungen der Formel (II) können z. B. durch Reaktion der entsprechenden Halogenverbindungen (IV) mit Boronsäuren oder Boronsäureestern des Typs (V) erhalten werden, wobei Y jeweils unabhängig voneinander z. B. H oder (C₁-C₆) Alkyl bzw. gemeinsam -C(CH₃)₂-C(CH₃)₂- oder -CH₂-C(CH₃)₂-CH₂- ist. Alternativ können auch Alkyl- oder Aryltrifluoroborate eingesetzt werden. Reaktionen dieses Typs sind ausführlich beschrieben z. B. in N. Miyaura, A. Suzuki, Chem. Rev. 1995, 95, 2457-2483, A. Suzuki, J. Organometallic Chem. 1999, 576, 147-168 oder G. A. Molander et al., J. Org. Chem. 2009, 74, 3626-3631.

Die Reaktion erfolgt im Allgemeinen unter Einsatz geeigneter Palladium-Quellen und Liganden wie z. B. Pd(PPh₃)₄, Pd(PPh₃)₂Cl₂, Pd(dppf)₂Cl₂, Pd(OAc)2 und XPhos, SPhos oder RuPhos. Als geeignete Basen werde z.B. Natriumhydrogencarbonat, Kaliumcarbonat, Kaliumphosphat, Caesiumfluorid oder Triethylamin eingesetzt. Die Reaktionen werden bei Temperaturen zwischen -20°C und 140°C durchgeführt, bevorzugt in Lösungsmitteln wie THF, DMF, Dioxan, Dimethoxyethan, Methanol oder Ethanol.

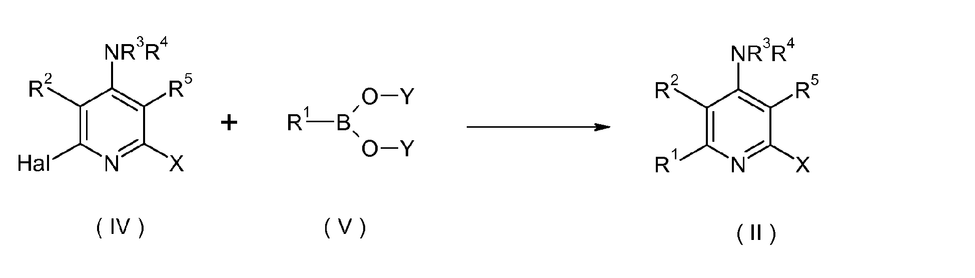

Die Synthese von Verbindungen der Formel (II), in welchen X ≠ Halogen ist (z. B. X = Methansulfonyl), sowie R² und/oder R⁵ ≠ Halogen ist, erfolgt aus den jeweiligen halogenierten Verbindungen (R² und/oder R⁵ = Halogen). So können z. B. mittels geeigneten Zinnreagenzien Alkyl- und Alkenylreste an diesen Stellen eingeführt werden, wie bereits an ähnlichen Substanzen beschrieben wurde (US 2009/0088322). Die Einführung von Alkinylresten kann z. B. durch Palladium katalysierte Reaktion mit entsprechenden terminalen Alkinen erfolgen (C. Saá et al., Tetrahedron 2006, 3843-3855).

Die Herstellung von Verbindungen der Formel (IV), in welchen z. B. X = Methansulfonyl ist, sind literaturbekannt (N. R. Pearson, T. S. Lardie, J. Labelled Cmpd. Radiopharm. 2006, 49, 965-972).

Die Herstellung von Verbindungen der Formel (V) erfolgt durch allgemein bekannte Methoden wie z. B. beschrieben in Boronic Acids: Preparation and Applications in Organic Synthesis and Medicine (D. G. Hall), Verlag Wiley, 2005.

Verbindungen der Formel (IV) können durch Halogenierung von (VI) mit geeigneten Reagenzien wie z. B. N-Iodosuccinimid, N-Bromosuccinimid, N-Chlorosuccinimid oder Selectfluor erhalten werden. Eine geeignete Reaktionsführung erlaubt dabei eine zeitgleiche doppelte Halogenierung (mit R² = R⁵), eine Schrittweise Einführung verschiedener Halogene (mit R² ≠ R⁵) oder die Einführung nur eines Halogens (mit R² oder R⁵ = H).

Die Reaktionen verlaufen dabei bevorzugt in organischen Lösungsmitteln wie z. B. Methanol, Ethanol, THF, Dioxan oder DMF bei Temperaturen zwischen 0°C und 120°C ab.

Entsprechende Nitrierungen von Verbindungen der Formel (VI) welche Verbindungen der Formel (IV) mit R² oder R⁵ = NO₂ ergeben, sind z. B. in WO 2006/31491 beschrieben. Die Synthese von Verbindungen der Formel (IV) mit R² oder R⁵ = CN wurden z. B. in Chemistry of Heterocyclic Compounds (S. v. Chapyshev), Vol. 27, 1991 beschrieben.

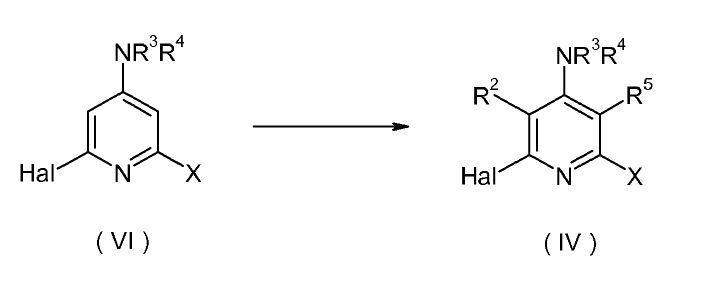

Verbindungen der Formel (VI) mit Resten R³ bzw. R⁴ ≠ H können z. B. durch Reaktionen der freien Amine mit verschiedenen Elektrophilen hergestellt werden. Entsprechende Beispiele findet man unter anderem in WO 2009/19553; WO 2008/107436; M. J. Ashton et al., J. Med. Chem. 1994, 37, 1696-1703; S. S. Laev et al., J. Fluorine Chem. 2001, 110, 43-46.

In den beschriebenen Formeln (II), (IV), (V) und (VI) haben die Reste R¹, R², R³, R⁴ und R⁵ jeweils die gleiche Bedeutung wie in Formel (I), Hal in den Formeln (IV) und (VI) bedeutet ein Halogenatom, X in den Formeln (II), (IV) und (VI) bedeutet jeweils ein Halogenatom oder einen Alkyl- / Arylsulfonyl-Rest.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, lpomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird. Obgleich die erfindungsgemäßen Verbindungen eine ausgezeichnete herbizide Aktivität gegenüber mono- und dikotylen Schadpflanzen aufweisen, werden Kulturpflanzen wirtschaftlich bedeutender Kulturen wie dikotyler Kulturen, z.B. der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen z.B. der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die erfindungsgemäßen Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die erfindungsgemäßen Verbindungen auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0 242 236 A, EP 0 242 246 A) oder Glyphosate (WO 92/000377 A) oder der Sulfonylharnstoffe (EP 0 257 993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0 142 924 A, EP 0 193 259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0 309 862 A, EP 0 464 461 A)
- gentechnisch veränderte Pflanzen mit reduzierter Photorespiration, die höhere Erträge und höhere Stresstoleranz aufweisen (EP 0 305 398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Verbindungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können z.B. in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser-und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streuund Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstoffformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequatchlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d . h . 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumicloracpentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosateisopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, Iodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamidedihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-31 0, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

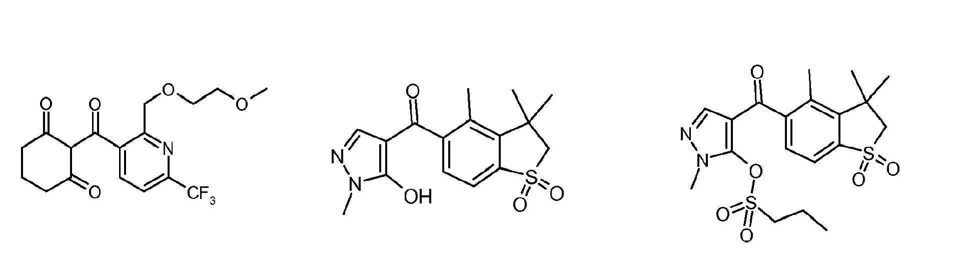

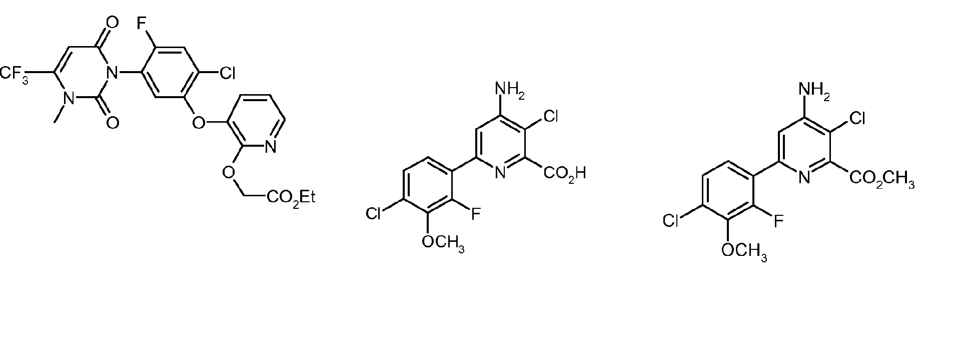

Besonders bevorzugt ist die Kombination des erfindungsgemäßen (4-Amino-3-chlor-6-(4-chlorphenyl)pyridin-2-yl)propandinitril mit einem weiteren Herbizid, ausgewählt aus der Gruppe bestehend aus Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequatchlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D , 2 , 4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1 H-tetrazol-1-yl]-phenyl]-ethansulfonamid, F-7967, d . h . 3-[7-Chlor-5-fluor-2-(trifluormethyl)-1H-benzimidazol-4-yl]-1-methyl-6-(trifluo-r methyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumicloracpentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosateisopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamidedihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-31 0, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl)anilin, sowie die folgenden Verbindungen: 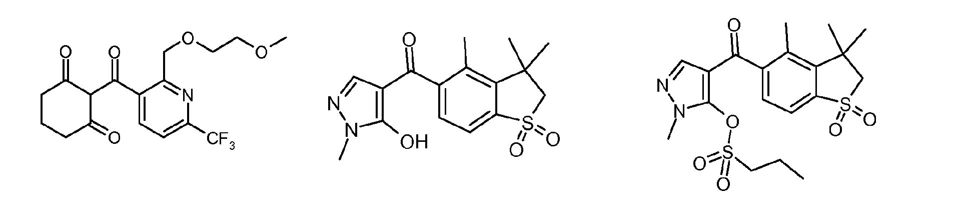 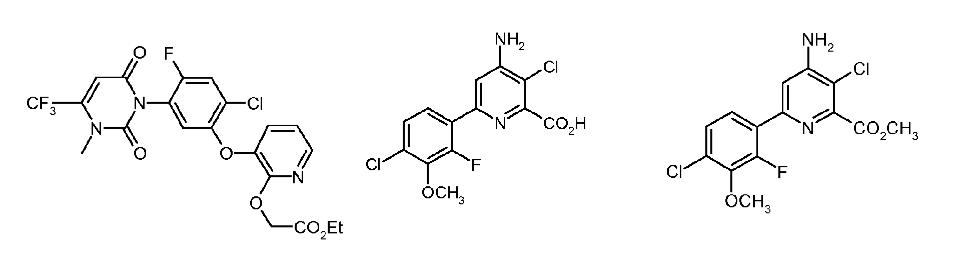 Von besonderem Interesse ist die selektive Bekämpfung von Schadpflanzen in Kulturen von Nutz- und Zierpflanzen. Obgleich die erfindungsgemäßen Verbindungen bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen von besonderem Interesse, welche die erfindungsgemäßen Verbindungen in Kombination mit Safenern enthalten, sowie gegebenenfalls weitere Pestizide wie Herbizide. Die Safener, welche in einem antidotisch wirksamen Gehalt eingesetzt werden, reduzieren die phytotoxischen Nebenwirkungen der eingesetzten Pestizide, z. B. in wirtschaftlich bedeutenden Kulturen wie Getreide (z.B. Weizen, Gerste, Roggen, Mais, Reis, Hirse), Zuckerrübe, Zuckerrohr, Raps, Baumwolle und Soja, vorzugsweise Getreide.

Folgende Gruppen von Verbindungen kommen beispielsweise als Safener in Frage:
S1) Verbindungen aus der Gruppe heterocyclischer Carbonsäurederivate:
   S1^{a}) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäure-ethylester (S1-4) und verwandte Verbindungen, wie sie in EP-A-333 131 und EP-A-269 806 beschrieben sind;
   S1^{c}) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäure-ethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1 H)-1,2,4-triazol-3-carbonsäuree thylester (S1-7), und verwandte Verbindungen, wie sie in EP-A-174 562 und EP-A-346 620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S 1-13), wie sie in der Patentanmeldung WO-A-95/07897 beschrieben sind.
   S2) Verbindungen aus der Gruppe der 8-Chinolinyloxyderivate (S2):
   S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocetmexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester r (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
   S3) Wirkstoffe vom Typ der Dichloracetamide (S3), die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" ( N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9)((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10), sowie dessen (R)-Isomer (S3-11).
   S4) Verbindungen aus der Klasse der Acylsulfonamide (S4):
   S4^{a}) N-Acylsulfonamide der Formel (S4^{a}) und deren Salze wie sie in der WO-A-97/45016 beschrieben sind, 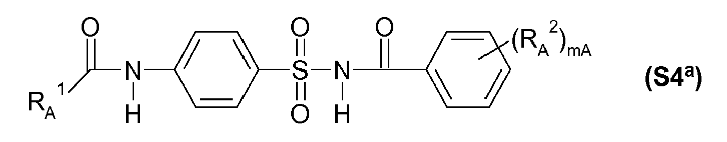 worin
      - R_{A}¹: (C₁-C₆)Alkyl, (C₃-C₆)Cycloalkyl, wobei die 2 letztgenannten Reste durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
      - R_{A}²: Halogen, (C₁₋C₄)Alkyl, (C₁₋C₄)Alkoxy, CF_{3;}
      - m_{A}: 1 oder 2;
      - v_{A}: ist 0, 1, 2 oder 3 bedeuten;
   S4^{b}) Verbindungen vom Typ der 4-(Benzoylsulfamoyl)benzamide der Formel (S4^{b}) und deren Salze, wie sie in der WO-A-99/16744 beschrieben sind, 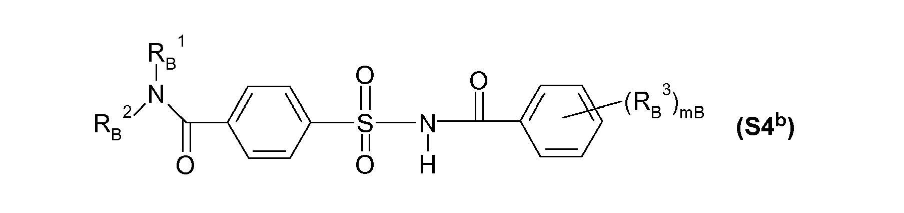 worin
      - R_{B}¹, R_{B}²: unabhängig voneinander Wasserstoff, (C₁-C₆)Alkyl, (C₃-C₆)Cycloalkyl, (C₃-C₆)Alkenyl, (C₃-C₆)Alkinyl,
      - R_{B}³: Halogen, (C₁-C₄)Alkyl, (C₁-C₄)Haloalkyl oder (C₁-C₄)Alkoxy und
      - m_{B}: 1 oder 2 bedeuten,
      z.B. solche worin
      R_{B}¹ = Cyclopropyl, R_{B}² = Wasserstoff und (R_{B}³) = 2-OMe ist (S4-1, "Cyprosulfamide",),
      R_{B}¹ = Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B}³) = 2-OMe ist (S4-3),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5).
   S4^{c}) Verbindungen aus der Klasse der Benzoylsulfamoylphenylharnstoffe der Formel (S4^{c}), wie sie in der EP-A-365484 beschrieben sind 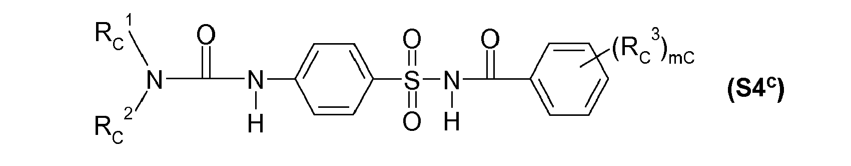 worin
      - R_{C}¹, R_{C}²: unabhängig voneinander Wasserstoff, (C₁-C₈)Alkyl, (C₃-C₈)Cycloalkyl, (C₃-C₆)Alkenyl, (C₃-C₆)Alkinyl,
      - R_{C}³: Halogen, (C₁-C₄)Alkyl, (C₁-C₄)Alkoxy, CF₃
      - m_{C}: 1 oder 2 bedeuten;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff.
   S5) Wirkstoffe aus der Klasse der Hydroxyaromaten und der aromatischaliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
   S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
   S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
   S8) Verbindungen der Formel (S8),wie sie in der WO-A-98/27049 beschrieben sind 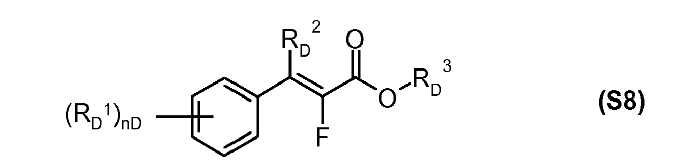 worin die Symbole und Indizes folgende Bedeutungen haben:
      - R_{D}¹: ist Halogen, (C₁-C₄)Alkyl, (C₁-C₄)Haloalkyl, (C₁-C₄)Alkoxy, (C₁-C₄)Haloalkoxy,
      - R_{D}²: ist Wasserstoff oder (C₁-C₄)Alkyl
      - R_{D}³: ist Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze
      - n_{D}: ist eine ganze Zahl von 0 bis 2.
   S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg. Nr.: 219479-18-2), 1,2-Dihydro-4-hydroxy-1-methyl-3-(5-tetrazolylcarbonyl)-2-chinolon (CAS-Reg.Nr. 95855-00-8), wie sie in der WO-A-1999/000020 beschrieben sind.
   S10) Verbindungen der Formeln (S10^{a}) oder (S10^{b}) wie sie in der WO-A-2007/023719 und WO-A-2007/023764 beschrieben sind 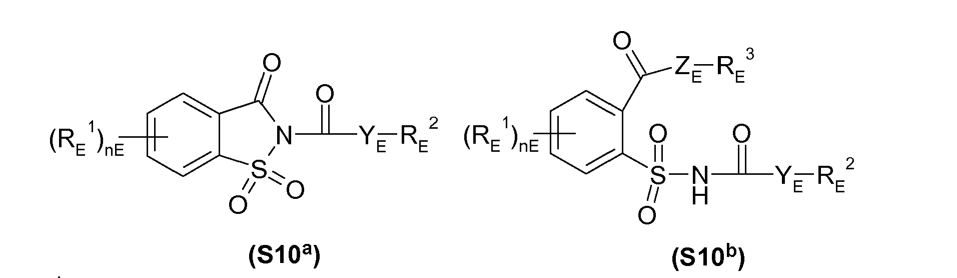 worin
      - RE¹: Halogen, (C₁-C₄)Alkyl, Methoxy, Nitro, Cyano, CF₃, OCF₃
      - Y_{E}, Z_{E}: unabhängig voneinander O oder S,
      - n_{E}: eine ganze Zahl von 0 bis 4,
      - R_{E}²: (C₁-C₁₆)Alkyl, (C₂-C₆)Alkenyl, (C₃-C₆)Cycloalkyl, Aryl; Benzyl, Halogenbenzyl,
      - R_{E}³: Wasserstoff oder (C₁-C₆)Alkyl bedeuten.
   S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino-(phenyl)acetonitril) (S11-1), das als Saatbeiz-Safener für Hirse gegen Schäden von Metolachlor bekannt ist,
      "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
      "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
   S12) Wirkstoffe aus der Klasse der Isothiochromanone (S12), wie z.B. Methyl-[(3-oxo-1 H-2-benzothiopyran-4(3H)-yliden)methoxy]acetat (CAS-Reg.Nr. 205121-04-6) (S12-1) und verwandte Verbindungen aus WO-A-1998/13361.
   S13) Eine oder mehrere Verbindungen aus Gruppe (S13):
      "Naphthalic anhydrid" (1,8-Naphthalindicarbonsäureanhydrid) (S13-1), das als Saatbeiz-Safener für Mais gegen Schäden von Thiocarbamatherbiziden bekannt ist,
      "Fenclorim" (4,6-Dichlor-2-phenylpyrimidin) (S13-2), das als Safener für Pretilachlor in gesätem Reis bekannt ist,
      "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
      "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
      "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
      "MG-8 3 8 " (CAS-Reg.Nr. 133993-74-5) (2-propenyl 1-oxa-4-azaspiro[4.5]decan-4-carbodithioat) (S13-6) der Firma Nitrokemia "Disulfoton" (O,O-Diethyl S-2-ethylthioethyl phosphordithioat) (S13-7),
      "Dietholate" (O,O-Diethyl-O-phenylphosphorothioat) (S13-8),
      "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
      "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "J C-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
      "Methoxyphenon" oder "N K 049" (3,3'-Dimethyl-4-methoxy-benzophenon),
   das als Safener für Reis gegen Schäden einiger Herbizide bekannt ist,
      "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
   S15) Verbindungen der Formel (S15) oder deren Tautomere wie sie in der WO-A-2008/131861 und WO-A-2008/131860 beschrieben sind 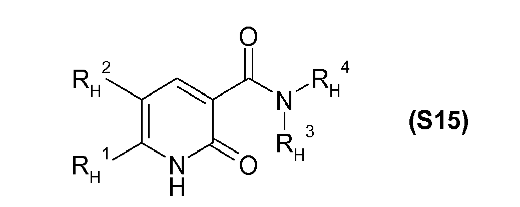 worin
      - R_{H}¹: einen (C₁-C₆)Haloalkylrest bedeutet und
      - R_{H}²: Wasserstoff oder Halogen bedeutet und
      - R_{H}³, R_{H}⁴: unabhängig voneinander Wasserstoff, (C₁-C₁₆)Alkyl, (C₂-C₁₆)Alkenyl oder (C₂-C₁₆)Alkinyl,
      wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
      oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
      wobei jeder der letztgenannten 4 Reste unsubstituiert oder durch einen oder mehrere Reste aus der Gruppe Halogen, Hydroxy, Cyano, (C1-C4)Alkyl, (C1-C4)Haloalkyl, (C1-C4)Alkoxy, (C1-C4)Haloalkoxy, (C1-C4)Alkylthio, (C1-C4)Alkylamino, Di[(C1-C4)alkyl]-amino, [(C1-C4)Alkoxy]-carbonyl, [(C1-C4)Haloalkoxy]-carbonyl, (C3-C6)Cycloalkyl, das unsubstituiert oder substituiert ist, Phenyl, das unsubstituiert oder substituiert ist, und Heterocyclyl, das unsubstituiert oder substituiert ist, substituiert ist,
      bedeutet oder
      - R_{H}³: (C₁-C₄)-Alkoxy, (C₂-C₄)Alkenyloxy, (C₂-C₆)Alkinyloxy oder (C₂-C₄)Haloalkoxy
      bedeutet und
      - R_{H}⁴: Wasserstoff oder (C₁-C₄)-Alkyl bedeutet oder
      - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
   S16) Wirkstoffe, die vorrangig als Herbizide eingesetzt werden, jedoch auch Safenerwirkung auf Kulturpflanzen aufweisen, z.B. (2,4-Dichlorphenoxy)essigsäure (2,4-D), (4-Chlorphenoxy)essigsäure, (R,S)-2-(4-Chlor-o-tolyloxy)propionsäure (Mecoprop), 4-(2,4-Dichlorphenoxy)buttersäure (2,4-DB), (4-Chlor-o-tolyloxy)essigsäure (MCPA), 4-(4-Chlor-o-tolyloxy)buttersäure, 4-(4-Chlorphenoxy)buttersäure, 3,6-Dichlor-2-methoxybenzoesäure (Dicamba),1-(Ethoxycarbonyl)ethyl-3,6-dichlor-2-methoxybenzoat (Lactidichlor-ethyl).

Einige der Safener sind auch als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den erfindungsgemäßen Verbindungen oder deren Mischungen mit weiteren Pestiziden formuliert werden und als Fertigformulierung oder Tankmischung mit den erfindungsgemäßen Verbindungen bereitgestellt und angewendet werden.

Besonders bevorzugt ist die Kombination des erfindungsgemäßen (4-Amino-3-chlor-6-(4-chlorphenyl)pyridin-2-yl)propandinitril mit wenigstens einem Safener, der ausgewählt ist aus den zuvor genannten Gruppen S1 bis S16.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der erfindungsgemäßen Verbindungen. Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

### Beispiele

### A. Synthesebeispiele

Nachfolgend sind Synthesen erfindungsgemäßer Verbindungen beispielhaft beschrieben, ohne dass diese Beispiele limitierenden Charakter haben.

### 1. (4-Amino-3-chlor-6-(4-chlorphenyl)pyridin-2-yl)propandinitril (Bsp. Nr. 43)

Zu einer Lösung von 1.00 g (3.97 mmol) 2,6-Dibrom-4-aminopyridin in 30 ml THF wurden 635 mg (4.76 mmol) N-Chlorsuccinimid gegeben. Die Reaktionsmischung wurde anschließend bei 50°C für 5 Stunden gerührt und nach dem Abkühlen auf 30 ml Wasser gegeben. Die wässrige Phase wurde mit 3x 30 ml Dichlormethan extrahiert, die vereinigten organischen Phasen über Magnesiumsulfat getrocknet und eingeengt. Nach der chromatographischen Reinigung des Rohprodukts über Kieselgel (n-Heptan/Dichlormethan 2:3) wurden 0.90 g (3.14 mmol, 79%) 2,6-Dibrom-3-chlorpyridin-4-amin erhalten.

Zu einer Lösung von 610 mg (2.13 mmol) 2,6-Dibrom-3-chlorpyridin-4-amin in 12 ml Toluol wurden unter Schutzgasatmosphäre 440 mg (2.81 mmol) 4-Chlorphenylboronsäure, 4 ml Natriumcarbonatlösung (2M in Wasser) und 35 mg (0.03 mmol) Pd(PPh₃)₄ gegeben. Die Mischung wurde in einem verschlossenen Glas für 2 Stunden bei 90°C in einem Mikrowellenreaktor erhitzt und nach dem Abkühlen auf 20 ml Wasser gegeben. Nach dem Extrahieren mit 3x 20 ml Dichlormethan wurden die vereinigten organischen Phasen über Magnesiumsulfat getrocknet und im Vakuum eingeengt. Nach Aufreinigung durch Säulenchromatographie über Kieselgel (n-Heptan/Dichlormethan 1:1) und Trennung der Regioisomere durch präparative HPLC konnten schließlich 135 mg (0.42 mmol, 20%) 2-Brom-3-chlor-6-(4-chlorphenyl)pyridin-4-amin erhalten.

Zu einer Lösung von 19 mg Malondinitril (0.29 mmol) in 2 ml Dimethoxyethan wurden unter Schutzgasatmosphäre 12 mg Natriumhydrid (0.3 mmol, 60%ige Dispersion in Mineralöl) zugegeben und für 10 Minuten gerührt. Anschließend wurden 60 mg (0.18 mmol) 2-Brom-3-chlor-6-(4-chlorphenyl)pyridin-4-amin und 23 mg (0.02 mmol) Pd(PPh₃)₄ zugegeben und die Mischung in einem verschlossenen Glas für 2 Stunden bei 100°C in einem Mikrowellenreaktor erhitzt. Nach dem Abkühlen wurden 10 ml Wasser hinzugefügt, die Reaktionsmischung mit wässriger Salzsäure (1N) angesäuert und mit 3x 10 ml Dichlormethan extrahiert. Nach dem Trocknen über Magnesiumsulfat, Einengen im Vakuum und Reinigung des Rohgemisches über präparative HPLC konnten 11 mg (0.036 mmol, 20%) (4-Amino-3-chlor-6-(4-chlorphenyl)pyridin-2-yl)propandinitril erhalten werden.

Die in den nachfolgenden Tabellen 1 - 4 beschriebenen Verbindungen erhält man analog zu den oben beschriebenen Synthesebeispielen.

In den Tabellen 1-4 bedeuten:

| | |
|---|---|
| Me | = Methyl |
| Et | = Ethyl |
| cBu | = Cyclobutyl |
| cPr | = Cyclopropyl |
| iPr | = Isopropyl |
| cHex | = Cyclohexyl |
| tBu | = tert-Butyl |
| Ph | = Phenyl |
| Vin | = Vinyl |
| Ac | = Acetyl |
| Hal | = Halogen |

**Tabelle 1: Verbindungen der Formel (I)**

| | | | | | |
|---|---|---|---|---|---|
| 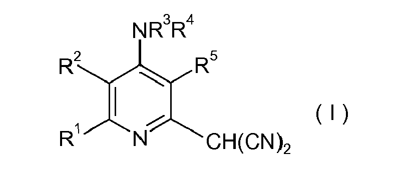 | | | | | |

| Bsp. Nr. | R¹ | R² | R³ | R⁴ | R⁵ |
|---|---|---|---|---|---|
| 1. | Ph | H | H | H | Cl |
| 2. | Ph | F | H | H | Cl |
| 3. | Ph | H | H | H | Br |
| 4. | Ph | F | H | H | Br |
| 5. | Ph | H | H | H | Me |
| 6. | Ph | F | H | H | Me |
| 7. | Ph | H | H | H | CH=CH₂ |
| 8. | Ph | F | H | H | CH=CH₂ |
| 9. | Ph | H | H | H | OMe |
| 10. | Ph | F | H | H | OMe |
| 11. | Ph | CN | H | H | Cl |
| 12. | Ph | Me | H | H | Cl |
| 13. | Ph | Cl | H | H | Cl |
| 14. | Ph | H | H | Me | Cl |
| 15. | Ph | H | Me | Me | Cl |
| 16. | Ph | H | H | Ac | Cl |
| 17. | Ph | F | H | Ac | Cl |
| 18. | 4-OMe-Ph | H | H | H | Cl |
| 19. | 4-OMe-Ph | H | H | H | Br |
| 20. | 4-NO₂-Ph | H | H | H | Cl |
| 21. | 4-NO₂-Ph | H | H | H | Br |
| 22. | 4-Me-Ph | H | H | H | F |
| 23. | 4-Me-Ph | H | H | H | Cl |
| 24. | 4-Me-Ph | H | H | H | Br |
| 25. | 4-I-Ph | H | H | H | Cl |
| 26. | 4-I-Ph | H | H | H | Br |
| 27. | 4-I-Ph | H | H | Ac | Cl |
| 28. | 4-I-Ph | H | H | Ac | Br |
| 29. | 4-I-Ph | H | H | Me | Cl |
| 30. | 4-I-Ph | H | H | Me | Br |
| 31. | 4-F-Ph | H | H | H | Cl |
| 32. | 4-F-Ph | H | H | H | Br |
| 33. | 4-F-Ph | H | H | Ac | Cl |
| 34. | 4-F-Ph | H | H | Ac | Br |
| 35. | 4-F-Ph | H | H | Me | Cl |
| 36. | 4-F-Ph | H | H | Me | Br |
| 37. | 4-COOMe-Ph | H | H | H | Cl |
| 38. | 4-COOMe-Ph | H | H | H | Br |
| 39. | 4-Cl-Ph | H | H | H | H |
| 40. | 4-Cl-Ph | Cl | H | H | H |
| 41. | 4-Cl-Ph | H | H | H | F |
| 42. | 4-Cl-Ph | F | H | H | F |
| 43. | 4-Cl-Ph | H | H | H | Cl |
| 44. | 4-Cl-Ph | F | H | H | Cl |
| 45. | 4-Cl-Ph | H | H | H | Br |
| 46. | 4-Cl-Ph | H | H | H | I |
| 47. | 4-Cl-Ph | H | H | H | CN |
| 48. | 4-Cl-Ph | Cl | H | H | Cl |
| 49. | 4-Cl-Ph | CN | H | H | Cl |
| 50. | 4-Cl-Ph | H | H | Me | Cl |
| 51. | 4-Cl-Ph | F | H | Me | Cl |
| 52. | 4-Cl-Ph | H | =CHN(Me)₂ | | Cl |
| 53. | 4-Cl-Ph | F | =CHN(Me)₂ | | Cl |
| 54. | 4-Cl-Ph | H | H | Ac | Cl |
| 55. | 4-Cl-Ph | H | H | Ac | Br |
| 56. | 4-Cl-Ph | H | H | Et | Cl |
| 57. | 4-Cl-Ph | H | H | Et | Br |
| 58. | 4-Cl-Ph | H | Me | Me | Cl |
| 59. | 4-Cl-Ph | H | Me | Me | Br |
| 60. | 4-CF3-Ph | H | H | H | Cl |
| 61. | 4-CF₃-Ph | H | H | H | Br |
| 62. | 4-CF3-Ph | H | H | Ac | Cl |
| 63. | 4-CF3-Ph | H | H | Ac | Br |
| 64. | 4-CF3-Ph | H | H | Me | Cl |
| 65. | 4-CF3-Ph | H | H | Me | Br |
| 66. | 4-Br-Ph | H | H | H | F |
| 67. | 4-Br-Ph | H | H | H | Cl |
| 68. | 4-Br-Ph | H | H | H | Br |
| 69. | 4-Br-Ph | H | H | H | I |
| 70. | 4-Br-Ph | H | H | H | CN |
| 71. | 4-Br-Ph | H | H | Ac | Cl |
| 72. | 4-Br-Ph | H | H | Ac | Br |
| 73. | 4-Br-Ph | H | H | Et | Cl |
| 74. | 4-Br-Ph | H | H | Et | Br |
| 75. | 4-Br-Ph | H | H | Me | Cl |
| 76. | 4-Br-Ph | H | H | Me | Br |
| 77. | 4-Br-Ph | H | Me | Me | Cl |
| 78. | 4-Br-Ph | H | Me | Me | Br |
| 79. | 3-OMe-4-F-Ph | H | H | H | F |
| 80. | 3-OMe-4-F-Ph | H | H | H | Cl |
| 81. | 3-OMe-4-F-Ph | H | H | H | Br |
| 82. | 3-OMe-4-Cl-Ph | H | H | H | F |
| 83. | 3-OMe-4-Cl-Ph | H | H | H | Cl |
| 84. | 3-OMe-4-Cl-Ph | H | H | H | Br |
| 85. | 3-Me-Ph | H | H | H | Cl |
| 86. | 3-Me-Ph | H | H | H | Br |
| 87. | 3-Me-Ph | H | H | H | F |
| 88. | 3-Me-4-Cl-Ph | H | H | H | F |
| 89. | 3-Me-4-Cl-Ph | H | H | H | Cl |
| 90. | 3-Me-4-Cl-Ph | H | H | H | Br |
| 91. | 3-F-Ph | H | H | H | F |
| 92. | 3-F-Ph | H | H | H | Cl |
| 93. | 3-F-Ph | H | H | H | Br |
| 94. | 3-Cl-Ph | H | H | H | Cl |
| 95. | 3-Cl-Ph | H | H | H | Br |
| 96. | 3-CF₃-Ph | H | H | H | F |
| 97. | 3-CF₃-Ph | H | H | H | Cl |
| 98. | 3-CF₃-Ph | H | H | H | Br |
| 99. | 3-CF₃-4-Cl-Ph | H | H | H | Cl |
| 100. | 3-CF₃-4-Cl-Ph | H | H | H | Br |
| 101. | 3-CF₃-4-Cl-Ph | H | H | H | F |
| 102. | 3-CF₃-4-Cl-Ph | H | H | H | I |
| 103. | 3,6-di-Cl-Ph | H | H | H | F |
| 104. | 3,6-di-Cl-Ph | H | H | H | Cl |
| 105. | 3,6-di-Cl-Ph | H | H | H | Br |
| 106. | 3,5-di-Cl-Ph | H | H | H | F |
| 107. | 3,5-di-Cl-Ph | H | H | H | Cl |
| 108. | 3,5-di-Cl-Ph | H | H | H | Br |
| 109. | 3,5-di-CF₃-Ph | H | H | H | F |
| 110. | 3,5-di-CF3-Ph | H | H | H | Cl |
| 111. | 3,5-di-CF3-Ph | H | H | H | Br |
| 112. | 3,4-di-O-Me-Ph | H | H | H | F |
| 113. | 3,4-di-OMe-Ph | H | H | H | Cl |
| 114. | 3,4-di-OMe-Ph | H | H | H | Br |
| 115. | 3,4-di-Cl-Ph | H | H | H | F |
| 116. | 3,4-di-Cl-Ph | H | H | H | Cl |
| 117. | 3,4-di-Cl-Ph | H | H | H | Br |
| 118. | 2-F-6-Cl-Ph | H | H | H | F |
| 119. | 2-F-6-Cl-Ph | H | H | H | Cl |
| 120. | 2-F-6-Cl-Ph | H | H | H | Br |
| 121. | 2-F-4-Cl-Ph | H | H | H | F |
| 122. | 2-F-4-Cl-Ph | H | H | H | Cl |
| 123. | 2-F-4-Cl-Ph | H | H | H | Br |
| 124. | 2-F-4-Cl-5-OMe-Ph | H | H | H | Cl |
| 125. | 2-F-4-Cl-5-OMe-Ph | H | H | H | Br |
| 126. | 2-F-4-Cl-5-OMe-Ph | H | H | Ac | Cl |
| 127. | 2_F-4-Cl-5-OMe-Ph | H | H | Ac | Br |
| 128. | 2-F-4-Cl-5-OMe-Ph | H | H | Me | Cl |
| 129. | 2-F-4-Cl-5-OMe-Ph | H | H | Me | Br |
| 130. | 2-F-4,5-di-Cl-Ph | H | H | H | F |
| 131. | 2-F-4,5-di-Cl-Ph | H | H | H | Cl |
| 132. | 2-F-4,5-di-Cl-Ph | H | H | H | Br |
| 133. | 2-F-3-SMe-4-Cl-Ph | H | H | H | Cl |
| 134. | 2-F-3-SMe-4-Cl-Ph | H | H | H | Br |
| 135. | 2-F-3-SMe-4-Cl-Ph | H | H | Ac | Cl |
| 136. | 2-F-3-SMe-4-Cl-Ph | H | H | Ac | Br |
| 137. | 2-F-3-SMe-4-Cl-Ph | H | H | Me | Cl |
| 138. | 2-F-3-SMe-4-Cl-Ph | H | H | Me | Br |
| 139. | 2-F-3-SCF₃-4-Cl-Ph | H | H | H | F |
| 140. | 2-F-3-SCF₃-4-Cl-Ph | H | H | H | Cl |
| 141. | 2-F-3-SCF₃-4-Cl-Ph | H | H | H | Br |
| 142. | 2-F-3-S(O₂)Me-4-Cl-Ph | H | H | H | F |
| 143. | 2-F-3-S(O₂)Me-4-Cl-Ph | H | H | H | Cl |
| 144. | 2-F-3-S(O₂)Me-4-Cl-Ph | H | H | H | Br |
| 145. | 2-F-3-S(O)Me-4-Cl-Ph | H | H | H | F |
| 146. | 2-F-3-S(O)Me-4-Cl-Ph | H | H | H | Cl |
| 147. | 2-F-3-S(O)Me-4-Cl-Ph | H | H | H | Br |
| 148. | 2-F-3-OMe-4-Cl-Ph | H | H | H | F |
| 149. | 2-F-3-OMe-4-Cl-Ph | H | H | H | Cl |
| 150. | 2-F-3-OMe-4-Cl-Ph | H | H | H | Br |
| 151. | 2-F-3-OMe-4-Cl-Ph | H | H | H | I |
| 152. | 2-F-3-OMe-4-Cl-Ph | H | H | H | CN |
| 153. | 2-F-3-OMe-4-Cl-Ph | F | H | H | Cl |
| 154. | 2-F-3-OMe-4-Cl-Ph | H | =CHN(Me)₂ | | Cl |
| 155. | 2-F-3-OMe-4-Cl-Ph | H | =CHN(Me)₂ | | Br |
| 156. | 2-F-3-OMe-4-Cl-Ph | H | H | Ac | Cl |
| 157. | 2-F-3-OMe-4-Cl-Ph | H | H | Ac | Br |
| 158. | 2-F-3-OMe-4-Cl-Ph | H | H | Et | Cl |
| 159. | 2-F-3-OMe-4-Cl-Ph | H | H | Et | Br |
| 160. | 2-F-3-OMe-4-Cl-Ph | H | H | Me | Cl |
| 161. | 2-F-3-OMe-4-Cl-Ph | H | H | Me | Br |
| 162. | 2-F-3-OMe-4-Cl-Ph | H | Me | Me | Cl |
| 163. | 2-F-3-OMe-4-Cl-Ph | H | Me | Me | Br |
| 164. | 2-F-3-OEt-4-Cl-Ph | H | H | H | Cl |
| 165. | 2-F-3-OEt-4-Cl-Ph | H | H | H | Br |
| 166. | 2-F-3-OEt-4-Cl-Ph | H | H | Ac | Cl |
| 167. | 2-F-3-OEt-4-Cl-Ph | H | H | Ac | Br |
| 168. | 2-F-3-OEt-4-Cl-Ph | H | H | Me | Cl |
| 169. | 2-F-3-OEt-4-Cl-Ph | H | H | Me | Br |
| 170. | 2-F-3-OCF₃-4-Cl-Ph | H | H | H | F |
| 171. | 2-F-3-OCF₃-4-Cl-Ph | H | H | H | Cl |
| 172. | 2-F-3-OCF₃-4-Cl-Ph | H | H | H | Br |
| 173. | 2-F-3-NMe₂-4-Cl-Ph | H | H | H | F |
| 174. | 2-F-3-NMe₂-4-Cl-Ph | H | H | H | Cl |
| 175. | 2-F-3-NMe₂-4-Cl-Ph | H | H | H | Br |
| 176. | 2-F-3-Me-4-Cl-Ph | H | H | H | F |
| 177. | 2-F-3-Me-4-Cl-Ph | H | H | H | Cl |
| 178. | 2-F-3-Me-4-Cl-Ph | H | H | H | Br |
| 179. | 2-Cl-Ph | H | H | H | F |
| 180. | 2-Cl-Ph | H | H | H | Cl |
| 181. | 2-Cl-Ph | H | H | H | Br |
| 182. | 2,6-di-F-3-OMe-4-Cl-Ph | H | H | H | F |
| 183. | 2,6-di-F-3-OMe-4-Cl-Ph | H | H | H | Cl |
| 184. | 2,6-di-F-3-OMe-4-Cl-Ph | H | H | H | Br |
| 185. | 2,6-di-Cl-Ph | H | H | H | F |
| 186. | 2,6-di-Cl-Ph | H | H | H | Cl |
| 187. | 2,6-di-Cl-Ph | H | H | H | Br |
| 188. | 2,5-di-Cl-Ph | H | H | H | F |
| 189. | 2,5-di-Cl-Ph | H | H | H | Cl |
| 190. | 2,5-di-Cl-Ph | H | H | H | Br |
| 191. | 2,4-di-F-5-OMe-Ph | H | H | H | F |
| 192. | 2,4-di-F-5-OMe-Ph | H | H | H | Cl |
| 193. | 2,4-di-F-5-OMe-Ph | H | H | H | Br |
| 194. | 2,4-di-F-3-OMe-Ph | H | H | H | F |
| 195. | 2,4-di-F-3-OMe-Ph | H | H | H | Cl |
| 196. | 2,4-di-F-3-OMe-Ph | H | H | H | Br |
| 197. | 2,4-di-Cl-Ph | H | H | H | F |
| 198. | 2,4-di-Cl-Ph | H | H | H | Cl |
| 199. | 2,4-di-Cl-Ph | H | H | H | Br |
| 200. | 2,4-di-Cl-Ph | H | H | Ac | F |
| 201. | 2,4-di-Cl-Ph | H | H | Ac | Cl |
| 202. | 2,4-di-Cl-Ph | H | H | Ac | Br |
| 203. | 2,4-di-Cl-5-F-Ph | H | H | H | F |
| 204. | 2,4-di-Cl-5-F-Ph | H | H | H | Cl |
| 205. | 2,4-di-Cl-5-F-Ph | H | H | H | Br |
| 206. | 2,4-di-Cl-3-OMe-Ph | H | H | H | F |
| 207. | 2,4-di-Cl-3-OMe-Ph | H | H | H | Cl |
| 208. | 2,4-di-Cl-3-OMe-Ph | H | H | H | Br |
| 209. | 2,4,6-tri-Cl-Ph | H | H | H | F |
| 210. | 2,4,6-tri-Cl-Ph | H | H | H | Cl |
| 211. | 2,4,6-tri-Cl-Ph | H | H | H | Br |

**Tabelle 2: Verbindungen der Formel (I)**

| | | | | | |
|---|---|---|---|---|---|
| 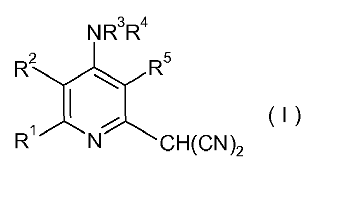 | | | | | |

| Bsp. Nr. | R¹ | R² | R³ | R⁴ | R⁵ |
|---|---|---|---|---|---|
| 212. | 1-Cl-cPr | H | H | H | F |
| 213. | 1-Cl-cPr | H | H | H | Cl |
| 214. | 1-Cl-cPr | H | H | H | Br |
| 215. | 1-Cl-cPr | F | H | H | F |
| 216. | 1-Cl-cPr | F | H | H | Cl |
| 217. | 1-Cl-cPr | H | H | Me | Cl |
| 218. | 1-Cl-cPr | H | Me | Me | Cl |
| 219. | 2-cPr-Vin | H | H | H | Cl |
| 220. | 2-cPr-Vin | F | H | H | Cl |
| 221. | 2-di-Me-cPr | H | H | H | Cl |
| 222. | 2-di-Me-cPr | F | H | H | Cl |
| 223. | 2-di-Me-cPr | H | H | H | F |
| 224. | 2-di-Me-cPr | F | H | H | F |
| 225. | 2-Me-Vin | H | H | H | Cl |
| 226. | 2-Me-Vin | F | H | H | Cl |
| 227. | 2-Ph-Vin | H | H | H | Cl |
| 228. | 2-Ph-Vin | F | H | H | Cl |
| 229. | 4-Cl-PhCH₂ | H | H | H | Cl |
| 230. | 4-Cl-PhCH₂ | F | H | H | Cl |
| 231. | cBu | F | H | H | Cl |
| 232. | cBu | Cl | H | H | Cl |
| 233. | cBu | H | H | H | Cl |
| 234. | CF₃ | F | H | H | Cl |
| 235. | CF₃ | Cl | H | H | Cl |
| 236. | CF₃ | H | H | H | Cl |
| 237. | cHex | F | H | H | Cl |
| 238. | cHex | Cl | H | H | Cl |
| 239. | cHex | H | H | H | Cl |
| 240. | cPr | F | H | H | Cl |
| 241. | cPr | Cl | H | H | Cl |
| 242. | cPr | H | H | H | Cl |
| 243. | cPr | H | H | H | F |
| 244. | cPr | H | H | H | Br |
| 245. | cPr | H | H | H | H |
| 246. | cPr | H | H | Ac | Cl |
| 247. | cPr | F | H | Ac | Cl |
| 248. | cPr | H | H | Et | Cl |
| 249. | cPr | F | H | Et | Cl |
| 250. | cPr | H | H | Me | Cl |
| 251. | cPr | F | H | Me | Cl |
| 252. | cPr | H | Me | Me | Cl |
| 253. | cPr | F | Me | Me | Cl |
| 254. | Hex | H | H | H | Cl |
| 255. | Hex | F | H | H | Cl |
| 256. | iPr | H | H | H | H |
| 257. | iPr | H | H | H | Cl |
| 258. | iPr | F | H | H | H |
| 259. | iPr | F | H | H | Cl |
| 260. | iPr | Cl | H | H | H |
| 261. | iPr | Cl | H | H | Cl |
| 262. | iPr | H | H | Ac | Cl |
| 263. | iPr | H | H | Me | Cl |
| 264. | Me | H | H | H | Cl |
| 265. | Me | F | H | H | Cl |
| 266. | tBu | H | H | H | Cl |
| 267. | tBu | F | H | H | Cl |
| 268. | tBu | Cl | H | H | Cl |
| 269. | tBu | Cl | H | H | H |
| 270. | Vin | H | H | H | Cl |
| 271. | Vin | F | H | H | Cl |
| 272. | Vin | Cl | H | H | Cl |

**Tabelle 3: Salze der Verbindungen der Formel (I)**

| | | | | | | |
|---|---|---|---|---|---|---|
| 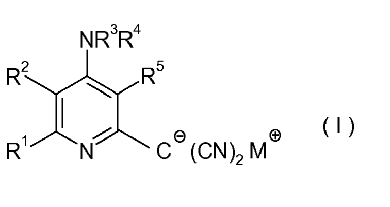 | | | | | | |

| Bsp. Nr. | R¹ | R² | R³ | R⁴ | R⁵ | M^{⊕} |
|---|---|---|---|---|---|---|
| 273. | Ph | H | H | H | Cl | Li⁺ |
| 274. | Ph | H | H | H | Cl | Na⁺ |
| 275. | Ph | H | H | H | Cl | K⁺ |
| 276. | Ph | H | H | H | Cl | Ca²⁺ |
| 277. | Ph | H | H | H | Cl | Mg²⁺ |
| 278. | Ph | H | H | H | Cl | NH₄⁺ |
| 279. | Ph | H | H | H | Cl | NH₃Me⁺ |
| 280. | Ph | H | H | H | Cl | NH2Me₂⁺ |
| 281. | Ph | H | H | H | Cl | NHMe₃⁺ |
| 282. | Ph | H | H | H | Cl | NMe₄⁺ |
| 283. | Ph | F | H | H | Cl | NH₄⁺ |
| 284. | Ph | F | H | H | Cl | Na⁺ |
| 285. | 4-OMe-Ph | H | H | H | Cl | NH₄⁺ |
| 286. | 4-NO₂-Ph | F | H | H | Cl | NH₄⁺ |
| 287. | 4-Me-Ph | H | H | H | Cl | NH₄⁺ |
| 288. | 4-1-Ph | F | H | H | Cl | NH₄⁺ |
| 289. | 4-F-Ph | H | H | H | Cl | Na⁺ |
| 290. | 4-F-Ph | F | H | H | Cl | K⁺ |
| 291. | 4-F-Ph | H | H | H | Cl | NH₄⁺ |
| 292. | 4-F-Ph | H | H | Ac | Cl | NH₄⁺ |
| 293. | 4-F-Ph | H | H | Me | Cl | NH₄⁺ |
| 294. | 4-Cl-Ph | H | H | H | Cl | Li⁺ |
| 295. | 4-Cl-Ph | H | H | H | Cl | Na⁺ |
| 296. | 4-Cl-Ph | H | H | H | Cl | K⁺ |
| 297. | 4-Cl-Ph | H | H | H | Cl | Ca²⁺ |
| 298. | 4-Cl-Ph | H | H | H | Cl | Mg²⁺ |
| 299. | 4-Cl-Ph | H | H | H | Cl | NH₄⁺ |
| 300. | 4-Cl-Ph | H | H | H | Cl | NH₃Me⁺ |
| 301. | 4-Cl-Ph | H | H | H | Cl | NH₂Me²⁺ |
| 302. | 4-Cl-Ph | H | H | H | Cl | NHMe₃⁺ |
| 303. | 4-Cl-Ph | H | H | H | Cl | NMe₄⁺ |
| 304. | 4-Cl-Ph | F | H | H | Cl | Na⁺ |
| 305. | 4-Cl-Ph | F | H | H | Cl | NH₄⁺ |
| 306. | 4-Cl-Ph | H | H | H | Br | Na⁺ |
| 307. | 4-Cl-Ph | H | H | H | Br | NH₄⁺ |
| 308. | 4-Cl-Ph | F | H | H | Br | NH₄⁺ |
| 309. | 4-Cl-Ph | F | H | H | Br | NH₄⁺ |
| 310. | 4-Cl-Ph | H | H | Ac | Cl | NH₄⁺ |
| 311. | 4-Cl-Ph | H | H | Et | Cl | NH₄⁺ |
| 312. | 4-Cl-Ph | H | H | Me | Cl | NH₄⁺ |
| 313. | 4-Cl-Ph | H | Me | Me | Cl | NH₄⁺ |
| 314. | 4-CF₃-Ph | H | H | H | Cl | NH₄⁺ |
| 315. | 4-Br-Ph | H | H | H | Cl | Na⁺ |
| 316. | 4-Br-Ph | H | H | H | Cl | K⁺ |
| 317. | 4-Br-Ph | H | H | H | Cl | NH₄⁺ |
| 318. | 4-Br-Ph | F | H | H | Cl | Na⁺ |
| 319. | 4-Br-Ph | F | H | H | Cl | K⁺ |
| 320. | 4-Br-Ph | F | H | H | Cl | NH₄⁺ |
| 321. | 4-Br-Ph | H | H | Ac | Cl | NH₄⁺ |
| 322. | 4-Br-Ph | H | H | Et | Cl | NH₄⁺ |
| 323. | 4-Br-Ph | H | H | Me | Cl | NH₄⁺ |
| 324. | 3-OMe-4-F-Ph | H | H | H | Cl | Na⁺ |
| 325. | 3-OMe-4-F-Ph | H | H | H | Cl | NH₄⁺ |
| 326. | 3-Me-Ph | F | H | H | Cl | NH₄⁺ |
| 327. | 3-Me-4-Cl-Ph | H | H | H | Cl | NH₄⁺ |
| 328. | 3-F-Ph | H | H | H | Cl | NH₄⁺ |
| 329. | 3-Cl-Ph | H | H | H | Cl | NH₄⁺ |
| 330. | 3-CF3-Ph | H | H | H | Cl | NH₄⁺ |
| 331. | 3-CF₃-4-Cl-Ph | H | H | H | Cl | NH₄⁺ |
| 332. | 3,6-di-Cl-Ph | H | H | H | Cl | NH₄⁺ |
| 333. | 3,5-di-Cl-Ph | H | H | H | Cl | NH₄⁺ |
| 334. | 3,5-di-CF3-Ph | H | H | H | Cl | NH₄⁺ |
| 335. | 3,4-di-OMe-Ph | H | H | H | Cl | NH₄⁺ |
| 336. | 3,4-di-Cl-Ph | H | H | H | Cl | Na⁺ |
| 337. | 3,4-di-Cl-Ph | H | H | H | Cl | NH₄⁺ |
| 338. | 2-F-6-Cl-Ph | H | H | H | Cl | NH₄⁺ |
| 339. | 2-F-4-Cl-Ph | H | H | H | Cl | Na⁺ |
| 340. | 2-F-4-Cl-Ph | H | H | H | Cl | K⁺ |
| 341. | 2-F-4-Cl-Ph | H | H | H | Cl | NH₄⁺ |
| 342. | 2-F-4-Cl-5-OMe-Ph | H | H | H | Cl | Na⁺ |
| 343. | 2-F-4-Cl-5-OMe-Ph | H | H | H | Cl | NH₄⁺ |
| 344. | 2-F-4-Cl-5-OMe-Ph | H | H | Ac | Cl | NH₄⁺ |
| 345. | 2-F-4,5-di-Cl-Ph | H | H | H | Cl | NH₄⁺ |
| 346. | 2-F-3-SMe-4-Cl-Ph | H | H | H | Cl | NH₄⁺ |
| 347. | 2-F-3-SCF₃-4-Cl-Ph | H | H | H | Cl | NH₄⁺ |
| 348. | 2-F-3-S(O₂)Me-4-Cl-Ph | H | H | H | Cl | NH₄⁺ |
| 349. | 2-F-3-S(O)Me-4-Cl-Ph | F | H | H | Cl | NH₄⁺ |
| 350. | 2-F-3-OMe-4-Cl-Ph | H | H | H | Cl | Li⁺ |
| 351. | 2-F-3-OMe-4-Cl-Ph | H | H | H | Cl | Na⁺ |
| 352. | 2-F-3-OMe-4-Cl-Ph | H | H | H | Cl | K⁺ |
| 353. | 2-F-3-OMe-4-Cl-Ph | H | H | H | Cl | Ca²⁺ |
| 354. | 2-F-3-OMe-4-Cl-Ph | H | H | H | Cl | Mg²⁺ |
| 355. | 2-F-3-OMe-4-Cl-Ph | H | H | H | Cl | NH₄⁺ |
| 356. | 2-F-3-OMe-4-Cl-Ph | H | H | H | Cl | NH₃Me⁺ |
| 357. | 2-F-3-OMe-4-Cl-Ph | H | H | H | Cl | NH₂Me₂⁺ |
| 358. | 2-F-3-OMe-4-Cl-Ph | H | H | H | Cl | NHMe₃⁺ |
| 359. | 2-F-3-OMe-4-Cl-Ph | H | H | H | Cl | NMe₄⁺ |
| 360. | 2-F-3-OMe-4-Cl-Ph | F | H | H | Cl | NH₄⁺ |
| 361. | 2-F-3-OMe-4-Cl-Ph | H | H | H | Br | NH₄⁺ |
| 362. | 2-F-3-OMe-4-Cl-Ph | H | H | H | OMe | NH₄⁺ |
| 363. | 2-F-3-OMe-4-Cl-Ph | H | H | H | CH=CH₂ | NH₄⁺ |
| 364. | 2-F-3-OMe-4-Cl-Ph | H | H | Ac | Cl | NH₄⁺ |
| 365. | 2-F-3-OMe-4-Cl-Ph | H | H | Me | Cl | NH₄⁺ |
| 366. | 2-F-3-OEt-4-Cl-Ph | H | H | H | Cl | NH₄⁺ |
| 367. | 2-F-3-OCF₃-4-Cl-Ph | H | H | H | Cl | NH₄⁺ |
| 368. | 2-F-3-NMe₂-4-Cl-Ph | H | H | H | Cl | NH₄⁺ |
| 369. | 2-Cl-Ph | H | H | H | Cl | NH₄⁺ |
| 370. | 2,6-di-F-3-OMe-4-Cl-Ph | H | H | H | Cl | NH₄⁺ |
| 371. | 2,6-di-Cl-Ph | H | H | H | Cl | NH₄⁺ |
| 372. | 2,5-di-Cl-Ph | H | H | H | Cl | NH₄⁺ |
| 373. | 2,4-di-F-5-OMe-Ph | H | H | H | Cl | NH₄⁺ |
| 374. | 2,4-di-F-3-OMe-Ph | H | H | H | Cl | NH₄⁺ |
| 375. | 2,4-di-Cl-Ph | H | H | H | Cl | NH₄⁺ |
| 376. | 2,4-di-Cl-Ph | F | H | H | Cl | NH₄⁺ |
| 377. | 2,4-di-Cl-5-F-Ph | H | H | H | Cl | NH₄⁺ |
| 378. | 2,4-di-Cl-3-OMe-Ph | H | H | H | Cl | NH₄⁺ |
| 379. | 2,4,6-tri-Cl-Ph | H | H | H | Cl | NH₄⁺ |

**Tabelle 4: Salze der Verbindungen der Formel (I)**

| | | | | | | |
|---|---|---|---|---|---|---|
| 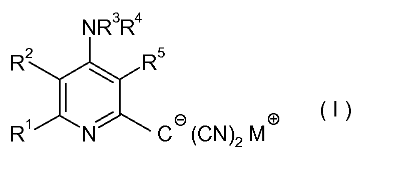 | | | | | | |

| Bsp. Nr. | R¹ | R² | R³ | R⁴ | R⁵ | M^{⊕} |
|---|---|---|---|---|---|---|
| 380. | 1-Cl-cPr | H | H | H | Cl | NH₄⁺ |
| 381. | 1-Cl-cPr | H | H | H | Cl | Li⁺ |
| 382. | 1-Cl-cPr | H | H | H | Cl | Na⁺ |
| 383. | 1-Cl-cPr | H | H | H | Cl | K⁺ |
| 384. | 1-Cl-cPr | H | H | H | Cl | Ca²⁺ |
| 385. | 1-Cl-cPr | H | H | H | Cl | Mg²⁺ |
| 386. | 1-Cl-cPr | H | H | H | Cl | NH₄⁺ |
| 387. | 1-Cl-cPr | H | H | H | Cl | NH₃Me⁺ |
| 388. | 1-Cl-cPr | H | H | H | Cl | NH₂Mₑ²⁺ |
| 389. | 1-Cl-cPr | H | H | H | Cl | NHMe₃⁺ |
| 390. | 1-Cl-cPr | H | H | H | Cl | NMe₄⁺ |
| 391. | 1-Cl-cPr | F | H | H | Cl | Na⁺ |
| 392. | 1-Cl-cPr | F | H | H | Cl | NH₄⁺ |
| 393. | 1-Cl-cPr | H | H | Me | Cl | NH₄⁺ |
| 394. | 1-Cl-cPr | H | H | Me | Cl | NH₄⁺ |
| 395. | 2-cPr-Vin | H | H | H | Cl | NH₄⁺ |
| 396. | 2-di-Me-cPr | H | H | H | Cl | Li⁺ |
| 397. | 2-di-Me-cPr | H | H | H | Cl | Na⁺ |
| 398. | 2-di-Me-cPr | H | H | H | Cl | K⁺ |
| 399. | 2-di-Me-cPr | H | H | H | Cl | Ca²⁺ |
| 400. | 2-di-Me-cPr | H | H | H | Cl | Mg²⁺ |
| 401. | 2-di-Me-cPr | H | H | H | Cl | NH₄⁺ |
| 402. | 2-di-Me-cPr | H | H | H | Cl | NH₃Me⁺ |
| 403. | 2-di-Me-cPr | H | H | H | Cl | NH₂Me₂⁺ |
| 404. | 2-di-Me-cPr | H | H | H | Cl | NHMe₃⁺ |
| 405. | 2-di-Me-cPr | H | H | H | Cl | NMe₄⁺ |
| 406. | 2-di-Me-cPr | F | H | H | Cl | NH₄⁺ |
| 407. | 2-Me-Vin | H | H | H | Cl | NH₄⁺ |
| 408. | 2-Ph-Vin | H | H | H | Cl | NH₄⁺ |
| 409. | 4-Cl-PhCH₂ | H | H | H | Cl | Na⁺ |
| 410. | 4-Cl-PhCH₂ | H | H | H | Cl | NH₄⁺ |
| 411. | cBu | H | H | H | Cl | Na⁺ |
| 412. | cBu | H | H | H | Cl | NH₄⁺ |
| 413. | CF₃ | H | H | H | Cl | NH₄⁺ |
| 414. | cHex | H | H | H | Cl | NH₄⁺ |
| 415. | cPr | H | H | H | Cl | Li⁺ |
| 416. | cPr | H | H | H | Cl | Na⁺ |
| 417. | cPr | H | H | H | Cl | K⁺ |
| 418. | cPr | H | H | H | Cl | Ca²⁺ |
| 419. | cPr | H | H | H | Cl | Mg²⁺ |
| 420. | cPr | H | H | H | Cl | NH₄⁺ |
| 421. | cPr | H | H | H | Cl | NH₃Me⁺ |
| 422. | cPr | H | H | H | Cl | NH₂Me²⁺ |
| 423. | cPr | H | H | H | Cl | NHMe₃⁺ |
| 424. | cPr | H | H | H | Cl | NMe₄⁺ |
| 425. | cPr | F | H | H | Cl | Na⁺ |
| 426. | cPr | F | H | H | Cl | NH₄⁺ |
| 427. | cPr | H | H | H | Br | NH₄⁺ |
| 428. | cPr | H | H | H | OMe | NH₄⁺ |
| 429. | cPr | H | H | Ac | Cl | Na⁺ |
| 430. | cPr | H | H | Ac | Cl | NH₄⁺ |
| 431. | cPr | H | H | Me | Cl | NH₄⁺ |
| 432. | cPr | H | Me | Me | Cl | NH₄⁺ |
| 433. | Hex | H | H | H | Cl | NH₄⁺ |
| 434. | iPr | H | H | H | Cl | Na⁺ |
| 435. | iPr | H | H | H | Cl | NH₄⁺ |
| 436. | iPr | F | H | H | Cl | NH₄⁺ |
| 437. | Me | H | H | H | Cl | NH₄⁺ |
| 438. | PhCH₂ | H | H | H | Cl | NH₄⁺ |
| 439. | PhCH₂ | H | H | H | Cl | Na⁺ |
| 440. | tBu | H | H | H | Cl | NH₄⁺ |
| 441. | tBu | F | H | H | Cl | NH₄⁺ |
| 442. | Vin | H | H | H | Cl | Na⁺ |
| 443. | Vin | H | H | H | Cl | NH₄⁺ |
| 444. | Vin | F | H | H | Cl | NH₄⁺ |

Physikalische Daten ausgewählter Verbindungen der Tabellen 1-4:

| Bsp. Nr. | Daten |
|---|---|
| 43. | 11.51 (bs, 1H), 7.96(bs, 1H), 7.66 (d, 2H), 7.59 (d, 2H), 7.32 (bs, 1 H), 6.51 (s, 1 H) |

Methode: ¹H-NMR (Bruker DRX-400, 400 MHz, 294K, DMSO-d₆, TMS = 0.0 ppm)

### B. Formulierungsbeispiele

a) Ein Stäubemittel wird erhalten, indem man 10 Gew.-Teile einer erfindungsgemäßen Verbindung und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer erfindungsgemäßen Verbindung, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer erfindungsgemäßen Verbindung mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer erfindungsgemäßen Verbindung, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer erfindungsgemäßen Verbindung,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer erfindungsgemäßen Verbindung
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 I/ha unter Zusatz von 0,2 Gew.-% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise hat die Verbindung Nr. 43 und andere Verbindungen aus den Tabellen 1 bis 4 sehr gute herbizide Wirkung von mindestens 80 % gegen Schadpflanzen wie Abuthilon theophrasti, Amaranthus retroflexus, Matricaria inodora, Polygonum (Fallopia) convolvulus und Veronica persica im Nachauflaufverfahren bei einer Aufwandmenge von 0.32 kg und weniger Aktivsubstanz pro Hektar. Gleichzeitig lassen erfindungsgemäße Verbindungen Gramineen-Kulturen wie Gerste, Weizen, Roggen, Hirse, Mais oder Reis im Nachauflaufverfahren selbst bei hohen Wirkstoffdosierungen ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. Pyridin-2-ylpropandinitrile der Formel (I), deren N-Oxide, Salze, und
agrochemisch geeigneten Derivate, 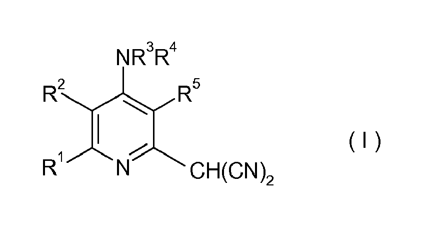 worin die Reste die folgende Bedeutung aufweisen:
R¹ ist Phenyl, optional substituiert mit einem, zwei oder drei Resten R^{x}, welche unabhängig voneinander ausgewält sind aus Halogen, Cyano, Nitro, Amino, (C₁-C₆)Alkyl, (C₁-C₆)Haloalkyl, (C₁-C₄)Hydroxyalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₂-C₄)Alkenyloxy, (C₂-C₄)Haloalkenyloxy, (C₂-C₄)Alkinyloxy, (C₃-C₄)Haloalkinyloxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, (C₂-C₄)Alkenylthio, (C₂-C₄)Haloalkenylthio, (C₂-C₄)Alkenylsulfinyl, (C₂-C₄)Haloalkenylsulfinyl, (C₂-C₄)Alkenylsulfonyl, (C₂-C₄)Haloalkenylsulfonyl, (C₂-C₄)Alkinylthio, (C₃-C₄)Haloalkinylthio, (C₃-C₄)Alkinylsulfinyl, (C₃-C₄)Haloalkinylsulfinyl, (C₃-C₄)Alkinylsulfonyl, (C₃-C₄)Haloalkinylsulfonyl, (C₁-C₆)Alkylamino, (C₂-C₈)Dialkylamino, (C₂-C₆)Alkylcarbonyl, (C₂-C₆)Alkoxycarbonyl, (C₂-C₆)Alkylaminocarbonyl, (C₃-C₈)Dialkylaminocarbonyl, (C₃-C₆)Trialkylsilyl, Phenyl, Phenoxy oder einen 5-oder 6-gliedrigen heteroaromatischen Ring, wobei jeder Phenyl-Ring, Phenoxy-Ring oder 5-oder 6-gliedrige heteroaromatische Ring optional substituiert sein kann mit einem, zwei oder drei Substituenten, die unabhängig voneinander ausgewählt sind aus R²⁸; oder wobei zwei benachbarte Reste R^{x} bilden gemeinsam eine -OCH₂O-, -CH₂CH₂O-, -OCH₂CH₂O-, -OCH(CH₃)O-, -OC(CH₃)₂O-, -OCF₂O-, -CF₂CF₂O-, -OCF₂CF₂O- oder - CH=CH-CH=CH- Gruppe bilden können; oder
(C₁-C₆)-Alkyl, optional substituiert mit einem, zwei oder drei Resten R^{y}, welche unabhängig voneinander ausgewählt sind aus Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio; oder
(C₂-C₆)-Alkenyl, optional substituiert mit einem, zwei oder drei Resten R^{z} welche unabhängig voneinander ausgewählt sind aus Halogen, (C₁-C₆)Alkyl, (C₁-C₆)Haloalkyl, (C₁-C₃)Alkoxy, (C₁-C₂)Haloalkoxy, (C₁-C₃)Alkylthio oder (C₁-C₂)Haloalkylthio;
R² ist ausgewählt aus Wasserstoff, Fluor, Chlor, Brom, Iod, Cyano, Nitro, Hydroxy (C₁-C₄)Alkyl, (C₂-C₄)Alkenyl, (C₂-C₄)Alkinyl, (C₁-C₄)Alkoxy, (C₁-C₄)Haloalkyl, (C₁-C₄)Haloalkoxy, SR⁶ oder N(R⁷)R⁸; worin R⁶ ausgewählt ist aus Wasserstoff, (C₁-C₄)Alkyl oder (C₁-C₃)Haloalkyl und R⁷ und R⁸ unabhängig voneinander ausgewählt sind aus Wasserstoff oder (C₁-C₄)Alkyl;
R³ ist ausgewählt aus Wasserstoff, (C₁-C₄)Alkyl optional substituiert mit einem oder zwei Resten R⁹, (C₂-C₄)Alkenyl optional substituiert mit einem oder zwei Resten R¹⁰, oder (C₂-C₄)Alkinyl optional substituiert mit einem oder zwei Resten R¹¹; oder R³ ist C(=O)R¹², NO₂, OR¹³, S(O)₂R¹⁴, N(R¹⁵)R¹⁶ oder N=C(R¹⁷)R¹⁸;
R⁴ ist ausgewählt aus Wasserstoff, (C₁-C₄)Alkyl optional substituiert mit einem oder zwei Resten R⁹, oder C(=O)R¹²;
oder
R³ und R⁴ bilden gemeinsam eine -(CH₂)₄-, -(CH₂)₅-, -CH₂CH=CHCH₂- oder -(CH₂)₂O(CH₂)₂- Gruppe, wobei jede der Gruppen optional substituiert sein kann mit einem oder zwei Resten R¹⁹; oder R³ und R⁴ gemeinsam eine =C(R²⁰)N(R²¹)R²² oder =C(R²³)OR²⁴ Gruppe bilden;
dabei ist jeder Rest R⁹, R¹⁰ und R¹¹ unabhängig voneinander Halogen, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
R⁵ ist ausgewählt aus Wasserstoff, Fluor, Chlor, Brom, Iod, Cyano, Nitro, Hydroxy, (C₁-C₄)Alkyl, (C₂-C₄)Alkenyl, (C₂-C₄)Alkinyl, (C₁-C₄)Alkoxy, (C₁-C₄)Haloalkyl, (C₁-C₄)Haloalkoxy, (C₁-C₄)Alkylthio, (C₁-C₄)Haloalkylthio, (C₁-C₄)Alkylamino, (C₁-C₄)Dialkylamino,
R¹² ist ggf. unabhängig von weiteren Resten R¹² ausgewählt aus Wasserstoff, (C₁-C₄)Alkyl, (C₁-C₃)Haloalkyl, (C₁-C₄)Alkoxy, Phenyl, Phenoxy oder Benzyloxy;
R¹³ ist Wasserstoff, (C₁-C₄)Alkyl, (C₁-C₃)Haloalkyl oder CHR²⁵C(O)OR²⁶;
R¹⁴ ist (C₁-C₄)Alkyl oder (C₁-C₃)Haloalkyl;
R¹⁵ ist Wasserstoff, (C₁-C₄)Alkyl oder C(=O)R²⁷;
R¹⁶ ist Wasserstoff oder (C₁-C₄)Alkyl;
R¹⁷ ist Wasserstoff, (C₁-C₄)Alkyl oder Phenyl optional substituiert mit einem, zwei oder drei Resten, welche voneinander unabhängig ausgewählt sind aus CH₃, Cl oder OCH₃;
R¹⁸ ist Wasserstoff oder (C₁-C₄)Alkyl, oder R¹⁷ und R¹⁸ bilden gemeinsam eine -(CH₂)₄- oder -(CH₂)₅- Gruppe;
R¹⁹ ggf. unabhängig von weiteren Resten R¹⁹ ausgewählt aus Halogen, (C₁-C₃)Alkyl, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
R²⁰ ist Wasserstoff oder (C₁-C₄)Alkyl;
R²¹ und R²² sind unabhängig voneinander ausgewählt aus Wasserstoff und (C₁-C₄)Alkyl, oder R²¹ und R²² bilden gemeinsam eine -(CH₂)₄-, -(CH₂)₅-, -CH₂CH=CHCH₂- oder -(CH₂)₂O(CH₂)₂- Gruppe;
R²³ ist Wasserstoff oder (C₁-C₄)Alkyl;
R²⁴ ist (C₁-C₄)Alkyl;
R²⁵ ist Wasserstoff, (C₁-C₄)Alkyl, (C₁-C₃)Haloalkyl, (C₁-C₄)Alkoxy, Phenyl, Phenoxy oder Benzyloxy;
R²⁶ ist Wasserstoff, (C₁-C₄)Alkyl oder (C₁-C₄)Alkoxy;
R²⁷ ist Wasserstoff, C₁-C₄ Alkyl oder Benzyl; und
R²⁸ ist ggf. unabhängig von weiteren Resten R²⁸ ausgewählt aus Halogen, Cyano, Nitro, (C₁-C₆)Alkyl, (C₁-C₆)Haloalkyl, (C₂-C₄)Alkenyl, (C₂-C₄)Haloalkenyl, (C₃-C₄)Alkinyl, (C₃-C₄)Haloalkinyl, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₄)Alkylamino, (C₂-C₈)Dialkylamino, (C₂-C₄)Alkylcarbonyl, (C₂-C₆)Alkoxycarbonyl, (C₂-C₆)Alkylaminocarbonyl, (C₃-C₈)Dialkylaminocarbonyl oder (C₃-C₆)Trialkylsilyl.

2. Pyridin-2-ylpropandinitrile der Formel (I) gemäß Anspruch 1, worin die Reste folgende Bedeutung aufweisen:
R¹ ist Phenyl, optional substituiert mit einem, zwei oder drei Resten R^{x}, welche unabhängig voneinander ausgewählt sind aus Halogen, Cyano, Nitro, Methyl, Ethyl, n-Propyl, i-Propyl, n-Butyl, t-Butyl, Cyclopropyl, Cyclobutyl, Cyclohexyl, , Difluormethyl, Trifluormethyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₆)Alkylamino, (C₂-C₈)Dialkylamino, oder wobei zwei benachbarte Reste R^{x} bilden gemeinsam eine -OCH₂O-, -CH₂CH₂O-, -OCH₂CH₂O-, -OCH(CH₃)O-, - OC(CH₃)₂O-, -OCF₂O-, -CF₂CF₂O- oder -OCF₂CF₂O- Gruppe bilden können; oder
(C₁-C₆)Alkyl, optional substituiert mit einem, zwei oder drei Resten R^{y}, welche unabhängig voneinander ausgewählt sind aus Fluor, Chlor, Brom, Iod, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio, oder
(C₂-C₆)Alkenyl, optional substituiert mit einem, zwei oder drei Resten R^{z}, welche unabhängig voneinander ausgewählt sind aus Fluor, Chlor, Brom, Iod" Methoxy, Ethoxy, n-Propoxy, i-Propoxy, n-Butoxy, t-Butoxy, (C₁-C₂)Haloalkoxy, (C₁-C₃)Alkylthio oder (C₁-C₂)Haloalkylthio,
R² ist Wasserstoff, Fluor, Chlor, Brom, Iod, Cyano, Methyl, Ethyl, Vinyl, Methoxy, Ethoxy;
R³ ist Wasserstoff, Methyl, Ethyl, n-Propyl, i-Propyl, n-Butyl, t-Butyl, (C₂-C₄)Alkenyl, (C₂-C₄)Alkinyl, C(=O)R¹², OR¹³, N(R¹⁵)R¹⁶ oder N=C(R¹⁷)R¹⁸;
R⁴ ist Wasserstoff oder Methyl, Ethyl, n-Propyl, i-Propyl, n-Butyl, t-Butyl, optional substituiert mit einem oder zwei Resten R⁹, oder C(=O)R¹²; oder
R³ und R⁴ bilden gemeinsam eine Gruppe -(CH₂)₄-, -(CH₂)₅-, -(CH₂)₂O(CH₂)₂- oder =C(R²⁰)N(R²¹)R²²;
dabei ist jeder Rest R⁹ unabhängig voneinander ausgewählt aus Fluor, Chlor, Brom, Iod" Methoxy, Ethoxy, n-Propoxy, i-Propoxy, Difluormethyl, Trifluormethyl" (C₁-C₃)Alkylthio, (C₁-C₃)Haloalkylthio, Amino, Methylamino, Ethylamino, n-Propylamino, i-Propylamino, Dimethylamino, Diethylamino, Methyl-Ethylamino oder (C₂-C₄)Alkoxycarbonyl;
R⁵ ist Wasserstoff, Fluor, Chlor, Brom, Iod, Cyano, Methyl, Ethyl, n-Propyl, i-Propyl, n-Butyl, t-Butyl Methoxy, Ethoxy, n-Propoxy, i-Propoxy, n-Butoxy, t-Butoxy, (C₁-C₄)Alkylthio,
R¹² ist Wasserstoff, Methyl, Ethyl, n-Propyl, i-Propyl, n-Butyl, t-Butyl, Difluormethyl, Trifluormethyl, Methoxy, Ethoxy, n-Propoxy, i-Propoxy, n-Butoxy, t-Butoxy, Phenyl, Phenoxy oder Benzyloxy;
R¹³ ist Wasserstoff, Methyl, Ethyl, n-Propyl, i-Propyl, n-Butyl, t-Butyl, Difluormethyl, Trifluormethyl, oder CHR²⁵C(O)OR²⁶;
R¹⁵ ist Wasserstoff, (C₁-C₄)Alkyl oder C(=O)R²⁷;
R¹⁶ ist Wasserstoff oder Methyl, Ethyl, n-Propyl, i-Propyl, n-Butyl, t-Butyl;
R¹⁷ ist Wasserstoff, Methyl, Ethyl, n-Propyl, i-Propyl, n-Butyl, t-Butyl oder Phenyl optional substituiert mit einem, zwei oder drei Resten, welche voneinander unabhängig ausgewählt sind aus CH₃, Cl oder OCH₃,
R¹⁸ ist Wasserstoff oder Methyl, Ethyl, n-Propyl, i-Propyl, n-Butyl, t-Butyl;
oder R¹⁷ und R¹⁸ bilden gemeinsam eine -(CH₂)₄- oder -(CH₂)₅- Gruppe;
R²⁰ ist Wasserstoff oder Methyl, Ethyl, n-Propyl, i-Propyl, n-Butyl, t-Butyl;
R²¹ und R²² sind unabhängig voneinander Wasserstoff oder Methyl, Ethyl, n-Propyl, i-Propyl, n-Butyl, t-Butyl; oder R²¹ and R²² bilden gemeinsam eine - (CH₂)₄-, -(CH₂)₅-, -CH₂CH=CHCH₂- oder -(CH₂)₂O(CH₂)₂- Gruppe;
R²⁵ ist Wasserstoff, Methyl, Ethyl, n-Propyl, i-Propyl, n-Butyl, t-Butyl, Difluormethyl, Trifluormethyl, (C₁-C₄)Alkoxy, Phenyl, Phenoxy oder Benzyloxy; und
R²⁶ ist Wasserstoff, Methyl, Ethyl, n-Propyl, i-Propyl, n-Butyl, t-Butyloder Methoxy, Ethoxy, n-Propoxy, i-Propoxy, n-Butoxy, t-Butoxy.

3. Pyridin-2-ylpropandinitrile der Formel (I) gemäß Anspruch 1 oder 2, worin die Reste folgende Bedeutung aufweisen:
R¹ ist Phenyl, optional substituiert mit einem, zwei oder drei Resten R^{x}, welche unabhängig voneinander ausgewählt sind aus Fluor, Chlor, Brom, Iod, Cyano, Nitro, Methylthio, Methyl, Ethyl oder wobei zwei benachbarte Reste R^{x} eine gemeinsame OCH₂O-, -OCH₂CH₂O- oder - OCH(CH₃)O- Gruppe bilden können;
oder Hexyl, Isopropyl, tert-Butyl, Cyclopropyl, Cyclobutyl, Cyclohexyl, Trifluormethyl, Trifluormethoxy, Methoxy, Ethoxy, (C₁-C₄)Haloalkoxy, Dimethylamino, Diethylamino, Dimethylamino, Trifluormethylsulfinyl, Methylsulfonyl, 4-Chlorbenzyl, Vinyl, Vinylphenyl, Cyclopropylvinyl, Methylvinyl;
R² ist Wasserstoff, Fluor, Chlor, Brom, Iod, Methyl, Ethyl, Vinyl, Methoxy oder Cyano;
R³ ist Wasserstoff, Methyl, Ethyl;
R⁴ ist Wasserstoff, Methyl, Ethyl oder Acetyl ; oder
R³ und R⁴ bilden gemeinsam eine =CHN(Me)₂ Gruppe
R⁵ ist Wasserstoff, F, Cl, Br, Methyl, Ethyl, Cyano, (C₁-C₄)Alkoxy, (C₁-C₄)Alkylthio.

4. (4-Amino-3-chlor-6-(4-chlorphenyl)pyridin-2-yl)propandinitril.

5. Agrochemisches Mittel, enthaltend a) mindestens eine Verbindung der Formel (I) oder deren N-Oxide, Salze und agrochemisch geeigneten Derivate, wie in einem oder mehreren der Ansprüche 1 bis 4 definiert, und b) im Pflanzenschutz übliche Hilfs- und Zusatzstoffe.

6. Agrochemisches Mittel, enthaltend a) mindestens eine Verbindung der Formel (I) oder deren N-Oxide, Salze und agrochemisch geeigneten Derivate, wie in einem oder mehreren der Ansprüche 1 bis 4 definiert, b) einen oder mehrere von Komponente a) verschiedene agrochemische Wirkstoffe, und optional c) im Pflanzenschutz übliche Hilfs- und Zusatzstoffe.

7. Verfahren zur Bekämpfung von unerwünschten Pflanzen oder zur Wachstumsregulierung von Pflanzen, wobei eine wirksame Menge mindestens einer Verbindung der Formel (I) oder deren N-Oxide, Salze und agrochemisch geeigneten Derivate, wie in einem oder mehreren der Ansprüche 1 bis 4 definiert, auf die Pflanzen, das Saatgut oder die Fläche, auf der die Pflanzen wachsen, aufgebracht wird.

8. Verwendung von Verbindungen der Formel (I) oder deren N-Oxide, Salze und agrochemisch geeigneten Derivate, wie in einem oder mehreren der Ansprüche 1 bis 4 definiert, als Herbizide oder Pflanzenwachstumsregulatoren.

9. Verwendung nach Anspruch 8, wobei die Verbindungen der Formel (I) oder deren N-Oxide, Salze und agrochemisch geeigneten Derivate zur Bekämpfung von Schadpflanzen oder zur Wachstumsregulierung in Pflanzenkulturen eingesetzt werden.

10. Verwendung nach Anspruch 9, wobei die Kulturpflanzen transgene oder nicht transgene Kulturpflanzen sind.

11. Verfahren zur Herstellung einer Verbindung der Formel (I) oder deren N-Oxide, Salze und agrochemisch geeigneten Derivate, wie in einem oder mehreren der Ansprüche 1 bis 4 definiert, wobei man
a) eine Verbindung der Formel (II) 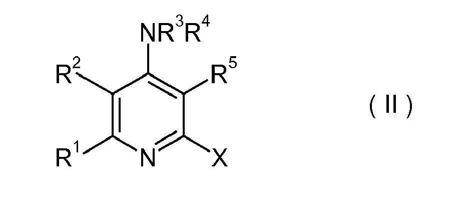 mit einem geeigneten Salz des Malondinitrils umsetzt und X eine geeignete Abgangsgruppe wie z.B. Chlor, Brom, Iod oder Methansulfonyl ist, die Reste R¹, R², R³, R⁴ und R⁵ in Formel (II) wie in Formel (I) in einem oder mehreren der Ansprüche 1 bis 3 definiert sind, und b) gegebenenfalls die in Schritt a) enthaltene Verbindung der Formel (I) nach üblichen Methoden in ein N-Oxid oder ein agrochemisch geeignetes Derivat überführt.

12. Verbindung der Formel (II), 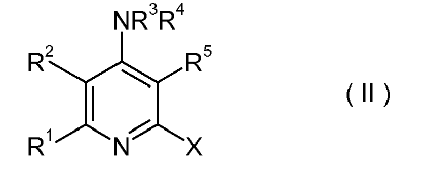 worin die Reste R¹, R², R³, R⁴ und R⁵ wie in Ansprüchen 1 bis 3 definiert sind.
